# EUROPÄISCHE PATENTSCHRIFT

(11) **EP 0 534 266 B1**
(45) Veröffentlichungstag und Bekanntmachung des Hinweises auf die Patenterteilung: **28.05.1997**
(21) Anmeldenummer: 92115698.0
(22) Anmeldetag: 14.09.1992
(51) Int. Cl.: C07D 249/12, A01N 47/38

(54) **Sulfonylaminocarbonyltriazolinone mit zwei über Sauerstoff gebundenen Substituenten**
Sulfonylaminocarbonyltriazolones with two through oxygen bonded substituents
Sulfonylaminocarbonyltriazolones ayant deux substituants liés à l'intermédiaire d'un oxygène

(30) Priorität: 25.09.1991 DE 4131842
(43) Veröffentlichungstag der Anmeldung: 31.03.1993
(73) Patentinhaber: BAYER AG, 51368 Leverkusen (DE)
(72) Erfinder: Haas, Wilhelm, Dr., W-5014 Kerpen 3 (DE); Müller, Klaus-Helmut, Dr., W-4000 Düsseldorf 13 (DE); König, Klaus, Dr., W-5068 Odenthal (DE); Santel, Hans-Joachim, Dr., W-5090 Leverkusen 1 (DE); Lürssen, Klaus, Dr., W-5060 Bergisch Gladbach 2 (DE); Schmidt, Robert R., Dr., W-5060 Bergisch Gladbach 2 (DE)

(56) Entgegenhaltungen:
- EP-A- 0 341 489
- EP-A- 0 422 469
- EP-A- 0 425 948
- EP-A- 0 431 291

## Beschreibung

Die Erfindung betrifft neue Sulfonylaminocarbonyltriazolinone mit zwei über Sauerstoff gebundenen Substituenten, mehrere Verfahren und neue Zwischenprodukte zu ihrer Herstellung sowie ihre Verwendung als Herbizide.

Es ist bekannt, daß bestimmte substituierte Sulfonylaminocarbonyltriazolinone, wie z.B. 2-(2-Chlor-phenylsulfonylaminocarbonyl)-4,5-dimethyl-2,4-dihydro-3H-1,2,4-triazol-3-on, herbizide Eigenschaften aufweisen (vgl. EP-A 341489; vgl. auch EP-A 422469, EP-A 425948 und EP-A 431291). Die Wirkung dieser Verbindungen ist jedoch nicht in allen Belangen zufriedenstellend.

Sulfonylaminocarbonyltriazolinone mit einem über Sauerstoff gebundenen Substituenten (in 5-Stellung) sind Gegenstand einer vorgängigen, jedoch nicht vorveröffentlichten Patentanmeldung (vgl. DE-P 4110795.O [LeA 28318] vom 04.04.1991).

Es wurden nun die neuen Sulfonylaminocarbonyltriazolinone mit zwei über Sauerstoff gebundenen Substituenten der allgemeinen Formel (I) 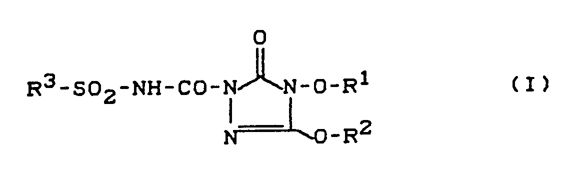 in welcher
- R¹: für Wasserstoff, für gegebenenfalls durch Fluor, Chlor, Cyano oder C₁-C₄-Alkoxy substituiertes C₁-C₆-Alkyl, für gegebenenfalls durch Fluor, Chlor und/oder Brom substituiertes C₃-C₆-Alkenyl oder für gegebenenfalls durch Fluor, Chlor, Brom und/oder C₁-C₄-Alkyl substituiertes C₃-C₇-Cycloalkyl steht,
- R²: für gegebenenfalls durch Fluor, Chlor, C₃-C₆-Cycloalkyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl oder für Cyclohexenyl steht und
- R³: für die Gruppierung 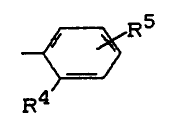 steht, worin
- R⁴ und R⁵: gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
- p: für die Zahlen 1 oder 2 steht und
- R⁶: für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
- R⁷: für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
- R⁴ und/oder R⁵: weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
- R⁸: für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
- R⁴ und/oder R⁵: weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
- R⁹: für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
- R³: für den Rest 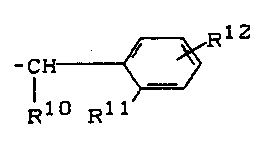 steht, worin
- R¹⁰: für Wasserstoff oder C₁-C₄-Alkyl steht,
- R¹¹ und R¹²: gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
- R³: für den Rest 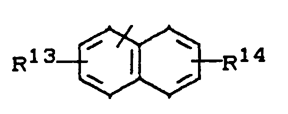 steht, worin
- R¹³ und R¹⁴: gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
- R³: für den Rest 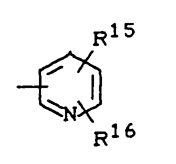 steht, worin
- R¹⁵ und R¹⁶: gleich oder verschieden sind und für Wasserstoff, Fluor, Chlor, Brom, Nitro, Cyano, C₁-C₄-Alkyl (welches gegebenenfalls durch Flu or und/oder Chlor substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsul fonyl (welche gegebenenfalls durch Fluor und/oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
- R³: für den Rest 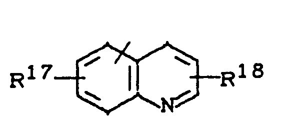 steht, worin
- R¹⁷ und R¹⁸: gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen;
weiterhin
- R³: für den Rest 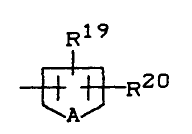 steht, worin
- R¹⁹ und R²⁰: gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
- A: für Sauerstoff, Schwefel oder die Gruppierung N-Z¹ steht, wobei
- Z¹: für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
- R³: für den Rest 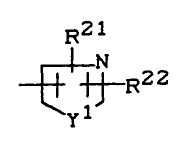 steht, worin
- R²¹ und R²²: gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
- Y¹: für Schwefel oder die Gruppierung N-R²³ steht, wobei
- R²³: für Wasserstoff oder C₁-C₄-Alkyl steht;
weiterhin
- R³: für den Rest 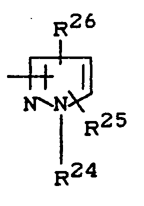 steht, worin
- R²⁴: für Wasserstoff, C₁-C₄-Alkyl, Benzyl, Pyridyl, Chinolinyl oder Phenyl steht,
- R²⁵: für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
- R²⁶: für Wasserstoff, Halogen oder C₁-C₄-Alkyl steht;
weiterhin
- R³: für eine der nachstehend aufgeführten Gruppierungen steht: 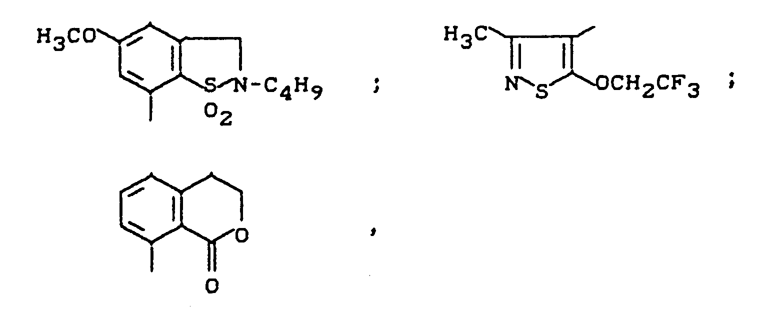
sowie Salze von Verbindungen der Formel (I) gefunden.

Man erhält die neuen Sulfonylaminocarbonyltriazolinone mit zwei über Sauerstoff gebundenen Substituenten der allgemeinen Formel (I), wenn man
(a) Triazolinone der allgemeinen Formel (II) 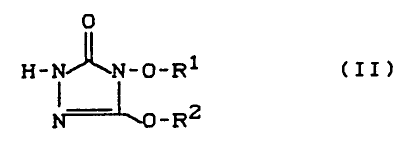 in welcher
   - R¹ und R²: die oben angegebenen Bedeutungen haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Verdünnungsmittels umsetzt, oder wenn man
(b) Triazolinon-Derivate der allgemeinen Formel (IV) 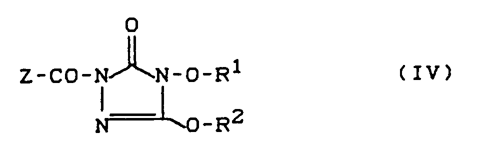 in welcher
   - R¹ und R²: die oben angegebenen Bedeutungen haben und
   - Z: für Chlor, C₁-C₄-Alkoxy, Benzyloxy oder Phenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt, oder wenn man
(c) Triazolinone der allgemeinen Formel (II) 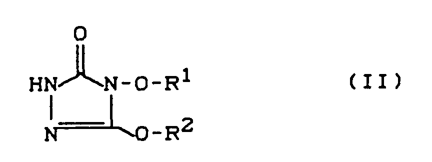 in welcher
   - R¹ und R²: die oben angegebenen Bedeutungen haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Chlor, C₁-C₄-Alkoxy, Benzyloxy oder Phenoxy steht,
   gegebenenfalls in Gegenwart eines Säureakzeptors und gegebenenfalls in Gegenwart eines Verdünnungsmittels umsetzt
und gegebenenfalls aus den nach Verfahren (a), (b) oder (c) hergestellten Verbindungen der Formel (I) nach üblichen Methoden Salze erzeugt.

Die neuen Sulfonylaminocarbonyltriazolinone mit zwei über Sauerstoff gebundenen Substituenten der allgemeinen Formel (I) und ihre Salze zeichnen sich durch starke herbizide Wirksamkeit aus.

Überraschenderweise zeigen die neuen Verbindungen der Formel (I) erheblich bessere herbizide Wirkung als die strukturell ähnliche bekannte Verbindung 2-(2-Chlorphenylsulfonylaminocarbonyl)-4,5-dimethyl-2,4-dihydro-3H-1,2,4-triazol-3-on.

Bevorzugte Salze sind insbesondere die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze von Verbindungen der Formel (I), in welcher R¹, R² und R³ die oben angegebenen Bedeutungen haben.

Gegenstand der Erfindung sind vorzugsweise Verbindungen der Formel (I), in welcher
- R¹: für Wasserstoff, für gegebenenfalls durch Fluor, Cyano, Methoxy oder Ethoxy substituiertes C₁-C₄-Alkyl, für Allyl oder für C₃-C₆-Cycloalkyl steht,
- R²: für gegebenenfalls durch Fluor und/oder Chlor, Methoxy oder Ethoxy substituiertes C₁-C₄-Alkyl, für gegebenenfalls durch Fluor und/oder Chlor substituiertes C₃-C₄-Alkenyl oder für C₃-C₆-Cycloalkyl steht, und
- R³: für die Gruppierung 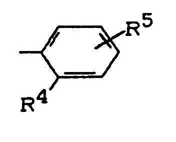 steht, worin
- R⁴: für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
- R⁵: für Wasserstoff, Fluor, Chlor oder Brom steht;
weiterhin
- R³: für den Rest 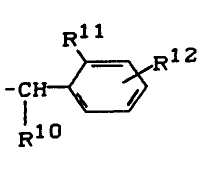 steht, worin
- R¹⁰: für Wasserstoff steht,
- R¹¹: für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
- R¹²: für Wasserstoff steht;
weiterhin
- R³: für den Rest 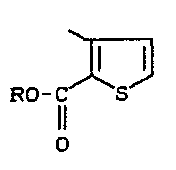 steht,
worin R für C₁-C₄-Alkyl steht, oder
für den Rest 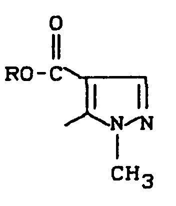 steht,
worin R für C₁-C₄-Alkyl steht.

Beispiele für die erfindungsgemäßen Verbindungen sind in der nachstehenden Tabelle 1 aufgeführt - vgl. auch die Herstellungsbeispiele.

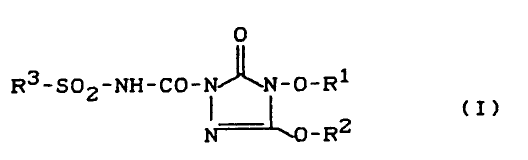

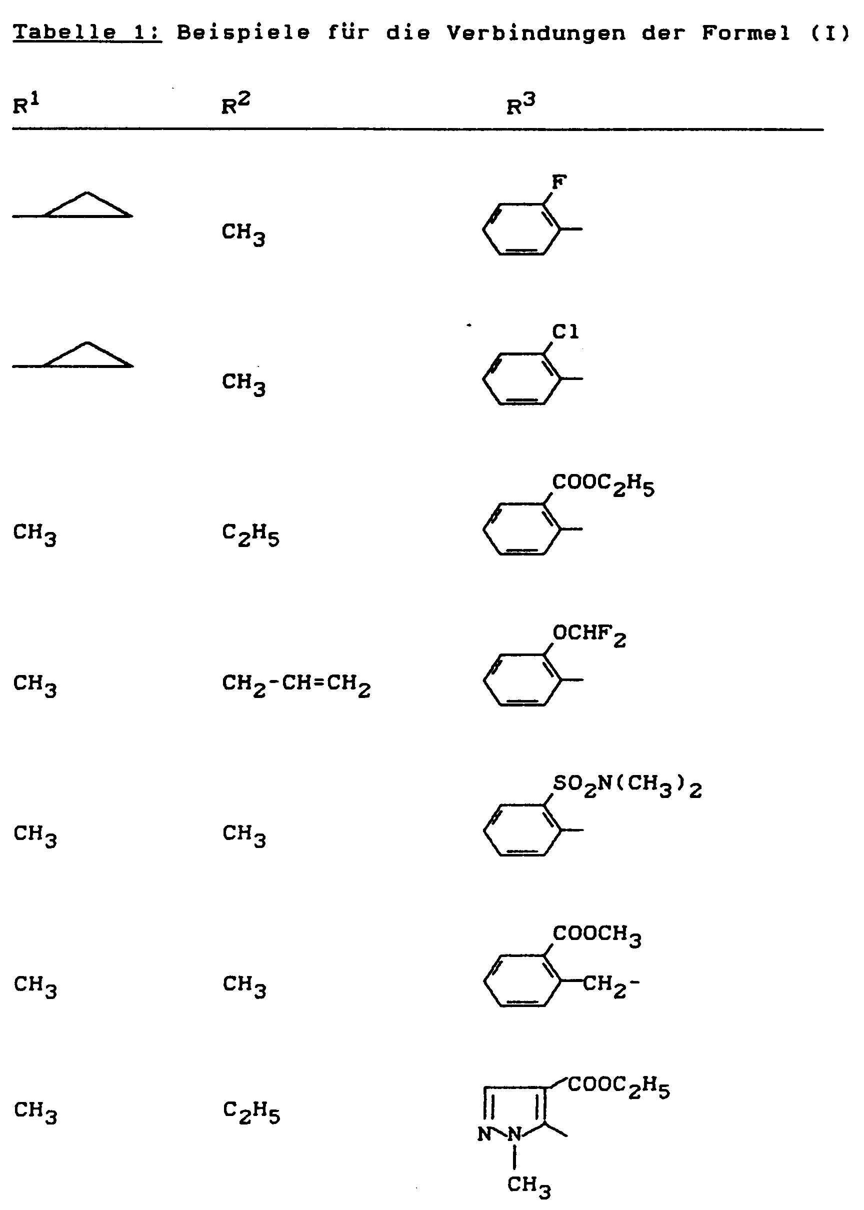

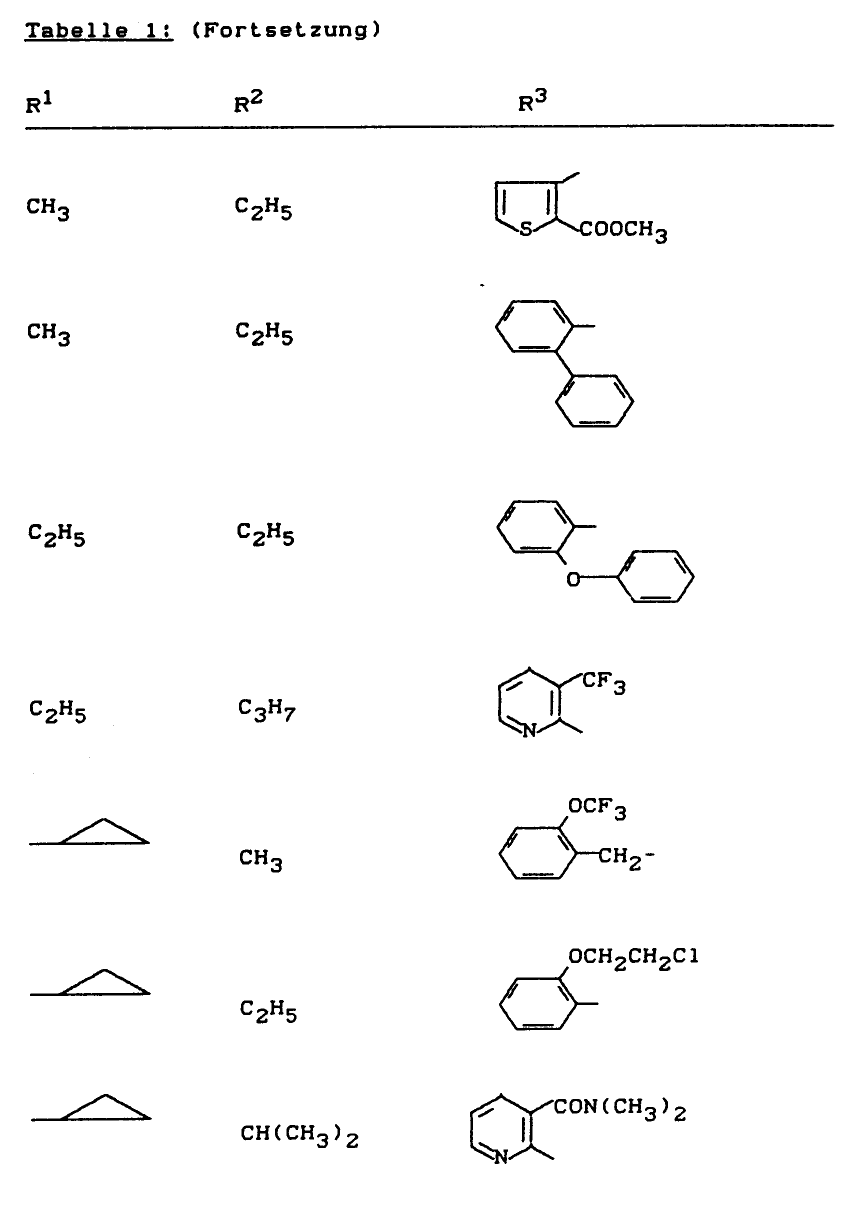

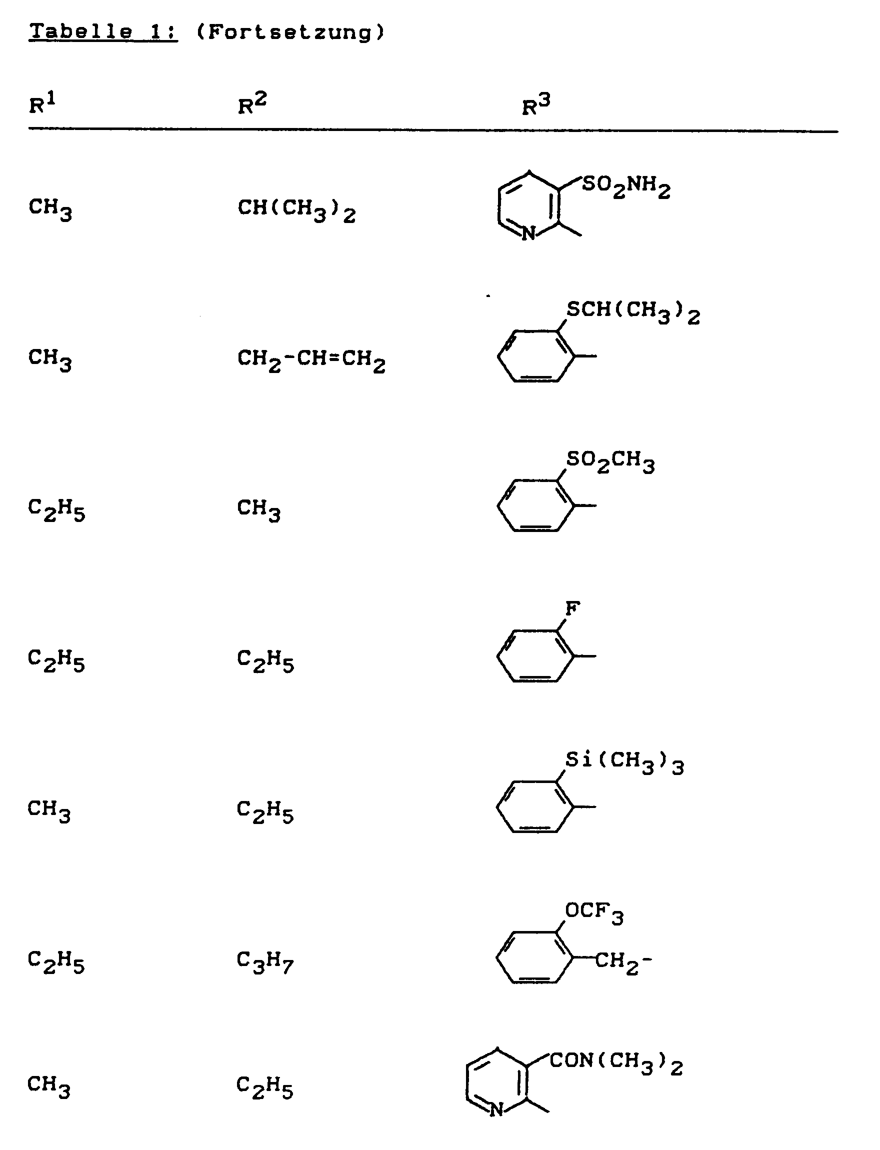

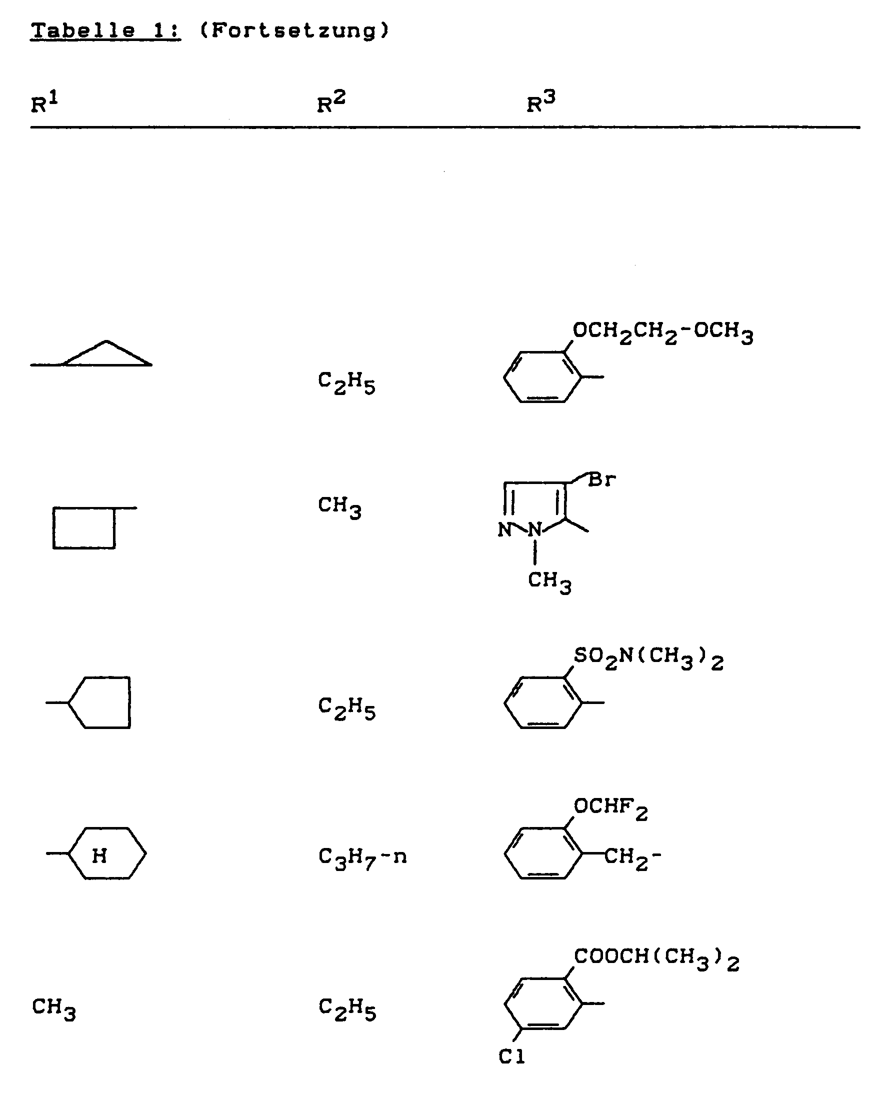

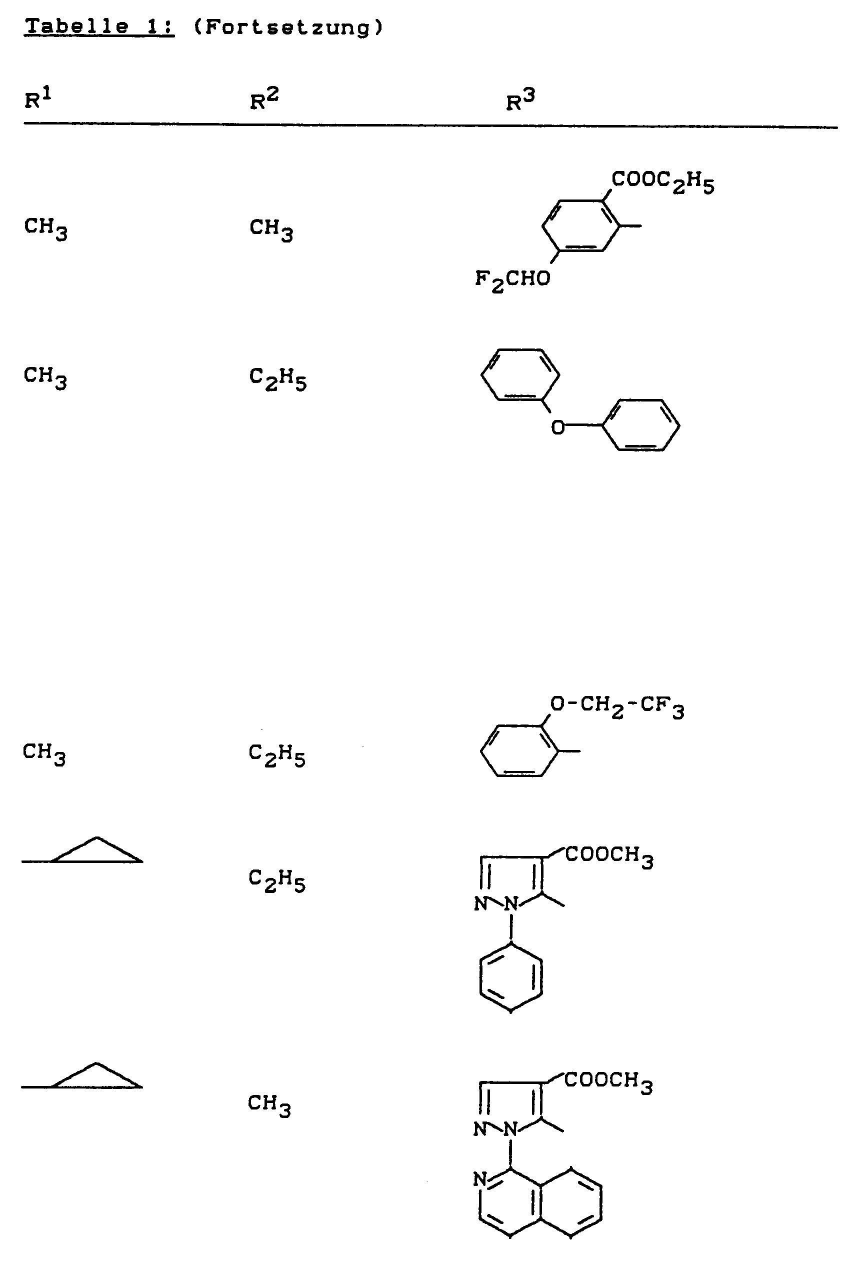

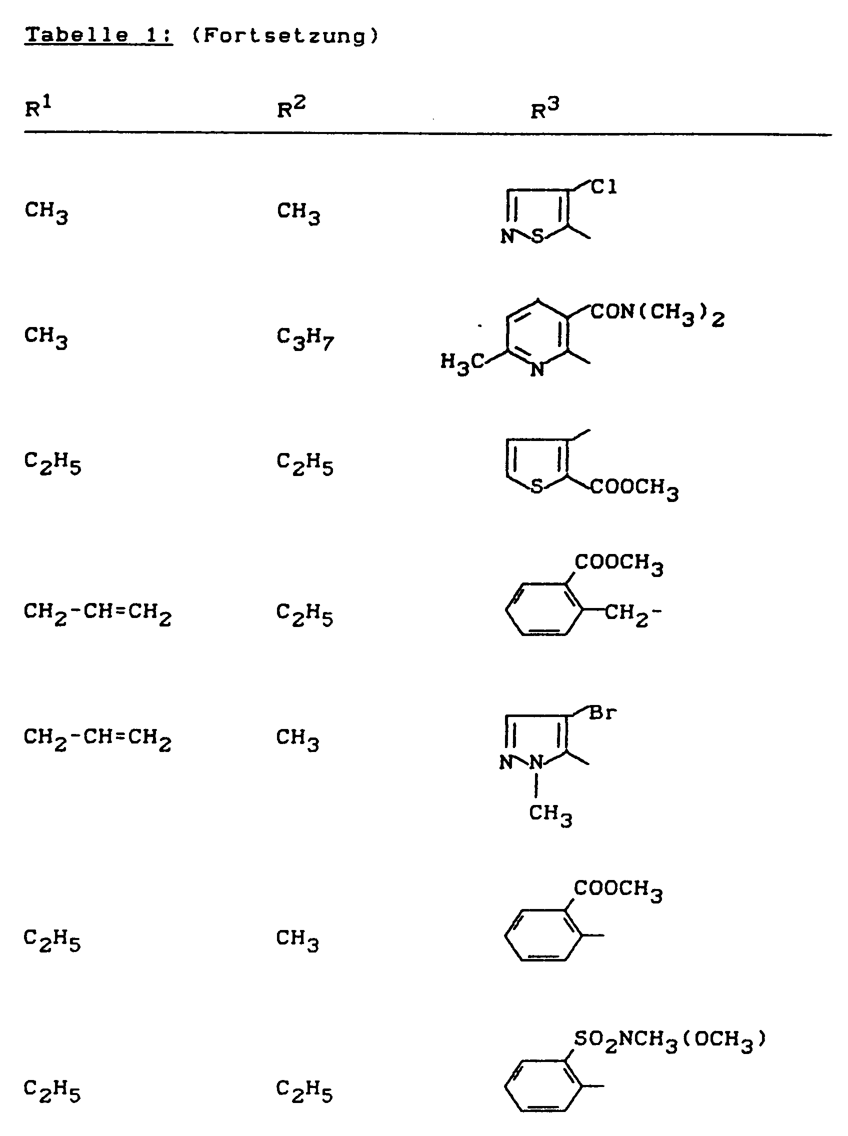

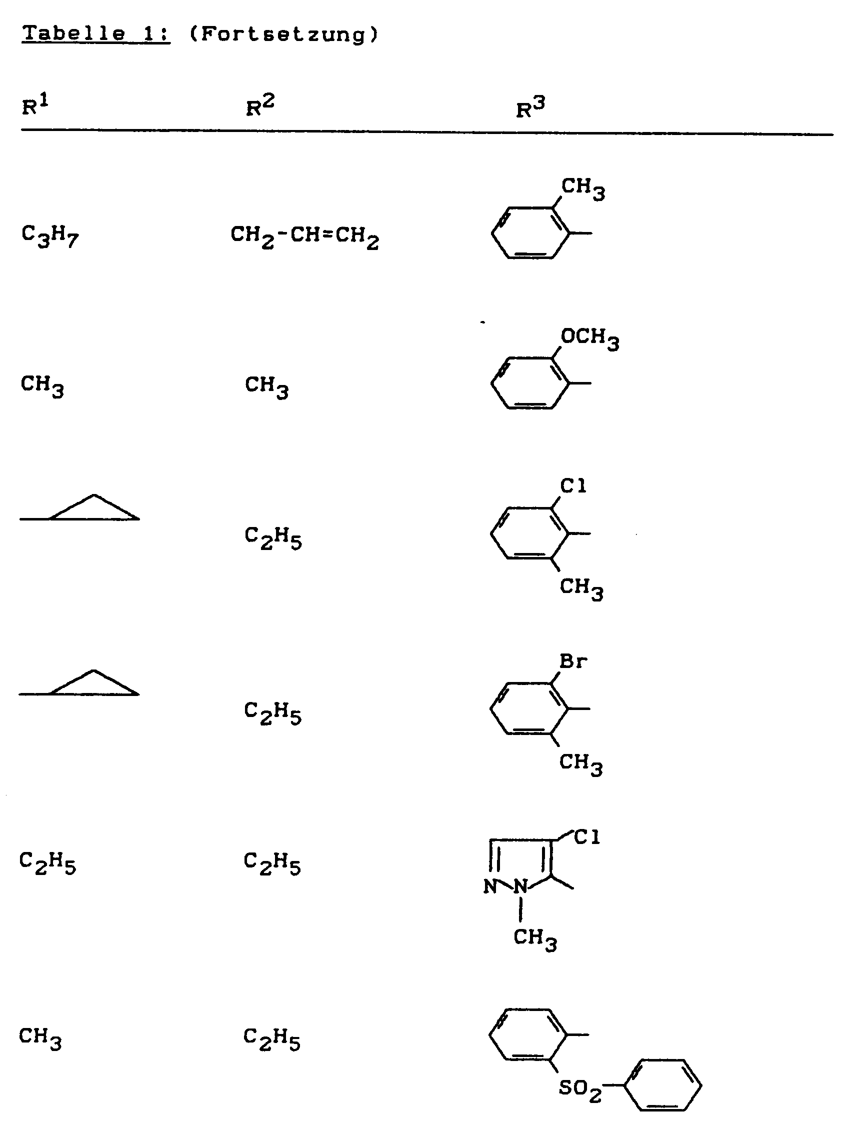

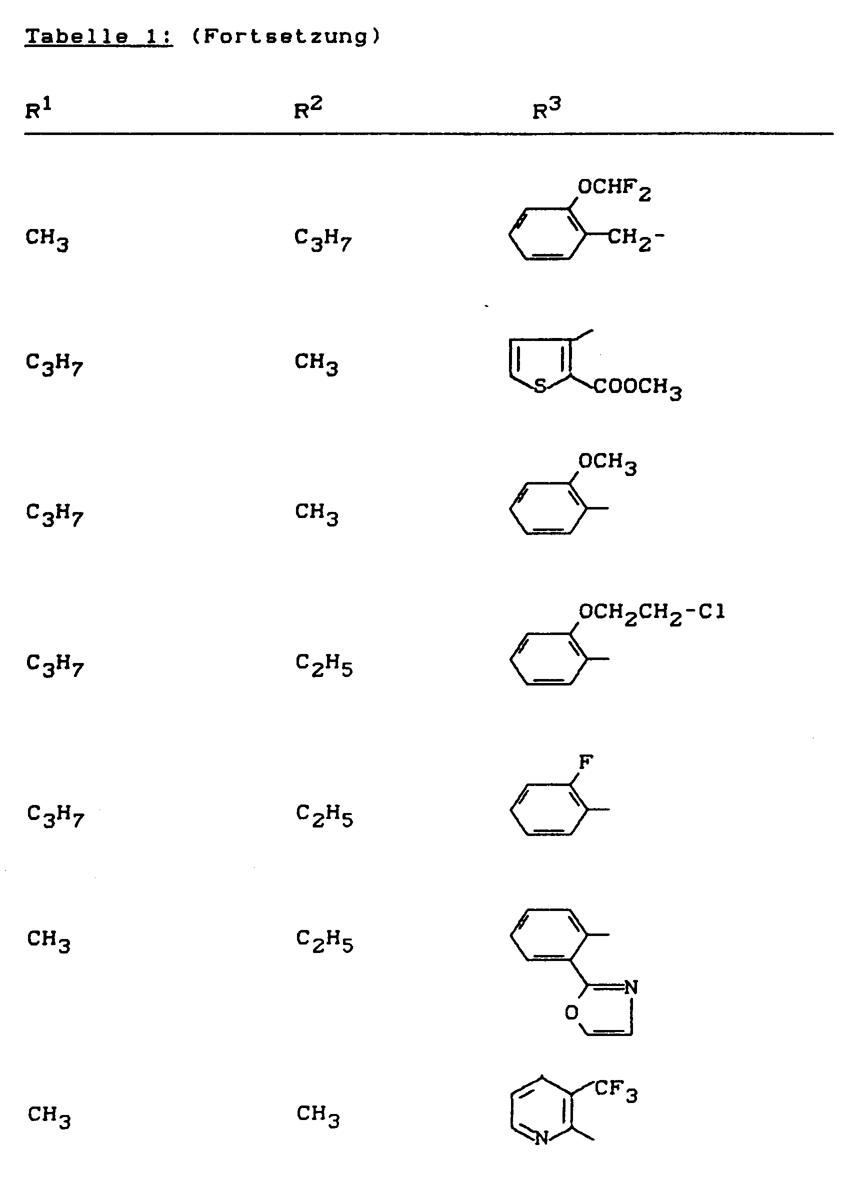

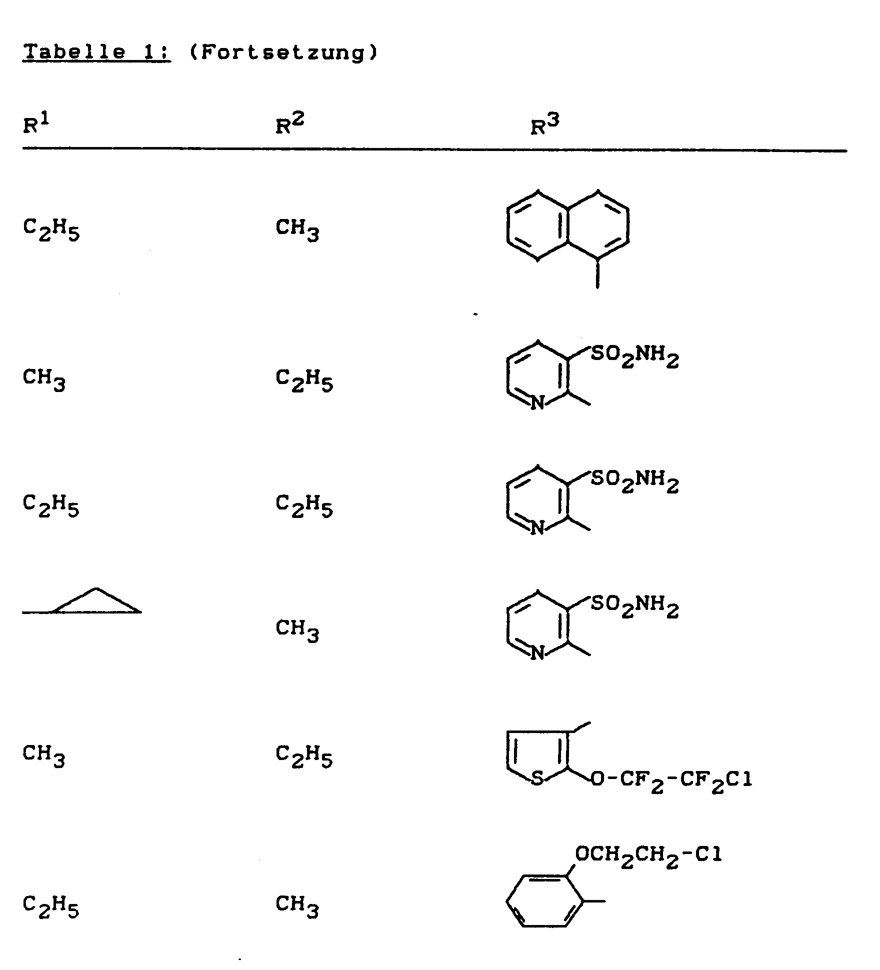

Verwendet man beispielsweise 2,6-Difluor-phenylsulfonylisocyanat und 5-Ethoxy-4-methoxy-2,4-dihydro-3H-1,2,4-triazol-3-on als Ausgangsstoffe, so kann der Reaktionsablauf beim erfindungsgemäßen Verfahren (a) durch folgendes Formelschema skizziert werden:

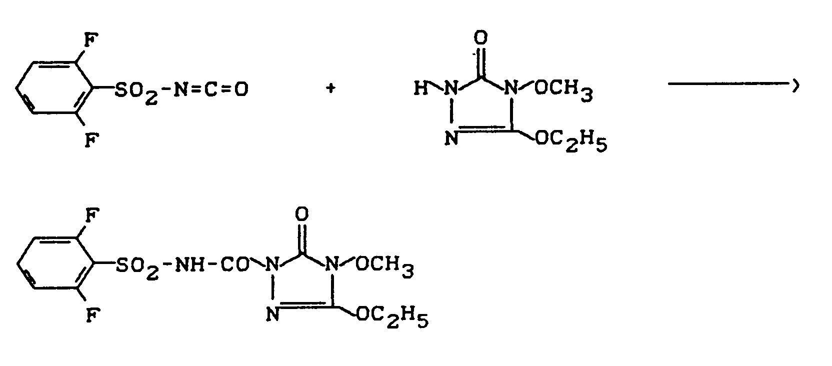

Verwendet man beispielsweise 2-Methylthio-benzolsulfonsäureamid und 2-Chlorcarbonyl-4-ethoxy-5-propyloxy-2,4-dihydro-3H-1,2,4-triazol-3-on als Ausgangsstoffe, so kann der Reaktionsablauf beim erfindungsgemäßen Verfahren (b) durch folgendes Formelschema skizziert werden:

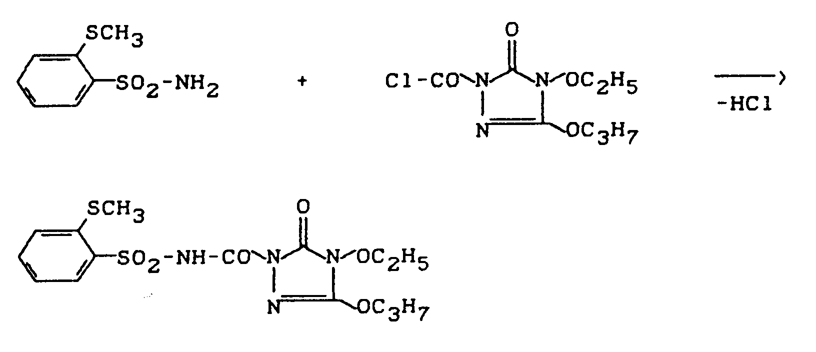

Verwendet man beispielsweise N-Methoxycarbonyl-2-methoxy-benzolsulfonsäureamid und 5-Methoxy-4-difluormethoxy-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

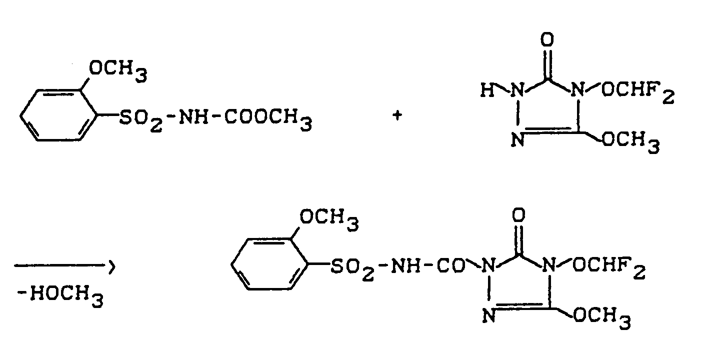

Die bei den erfindungsgemäßen Verfahren (a) und (c) zur Herstellung von Verbindungen der Formel (I) als Ausgangsstoffe zu verwendenden Triazolinone sind durch die Formel (II) allgemein definiert.

In Formel (II) haben R¹ und R² vorzugsweise diejenigen Bedeutungen, die bereits oben im Zusammenhang mit der Beschreibung der erfindungsgemäßen Verbindungen der Formel (I) vorzugsweise für R¹ und R² angegeben wurden.

Beispiele für die Ausgangsstoffe der Formel (II) sind in der nachstehenden Tabelle 2 aufgeführt.

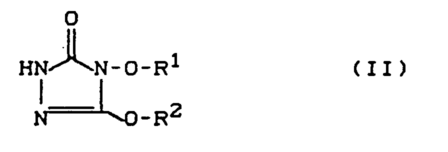

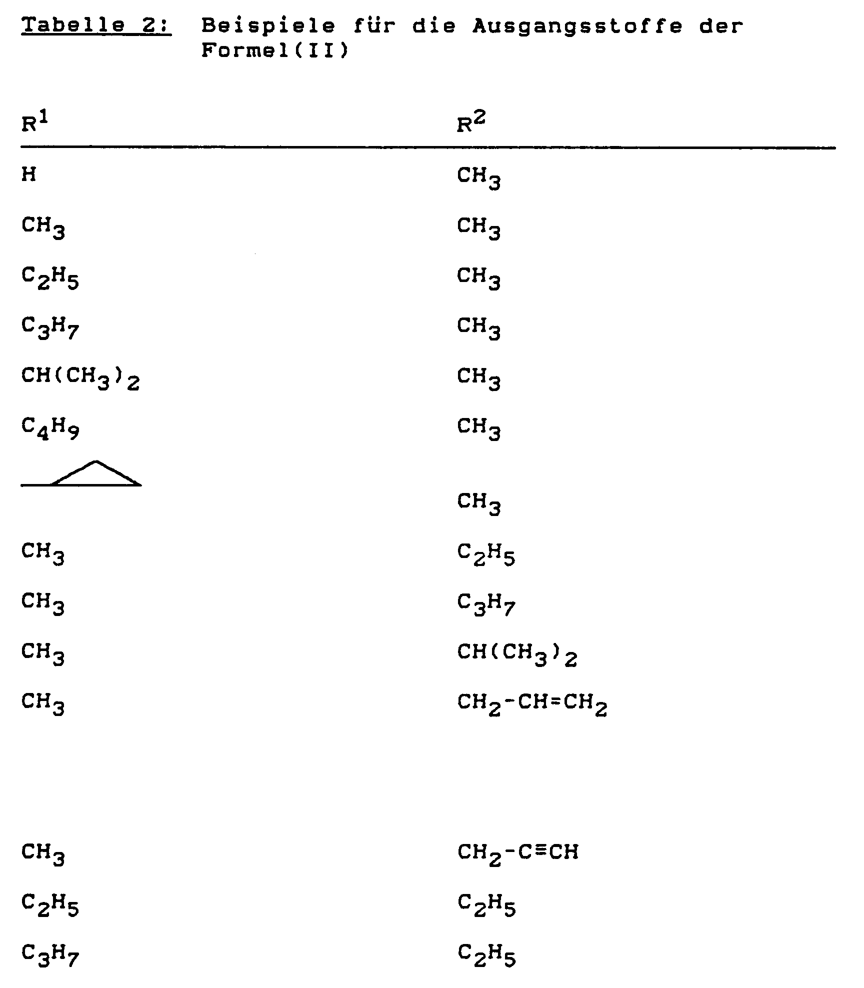

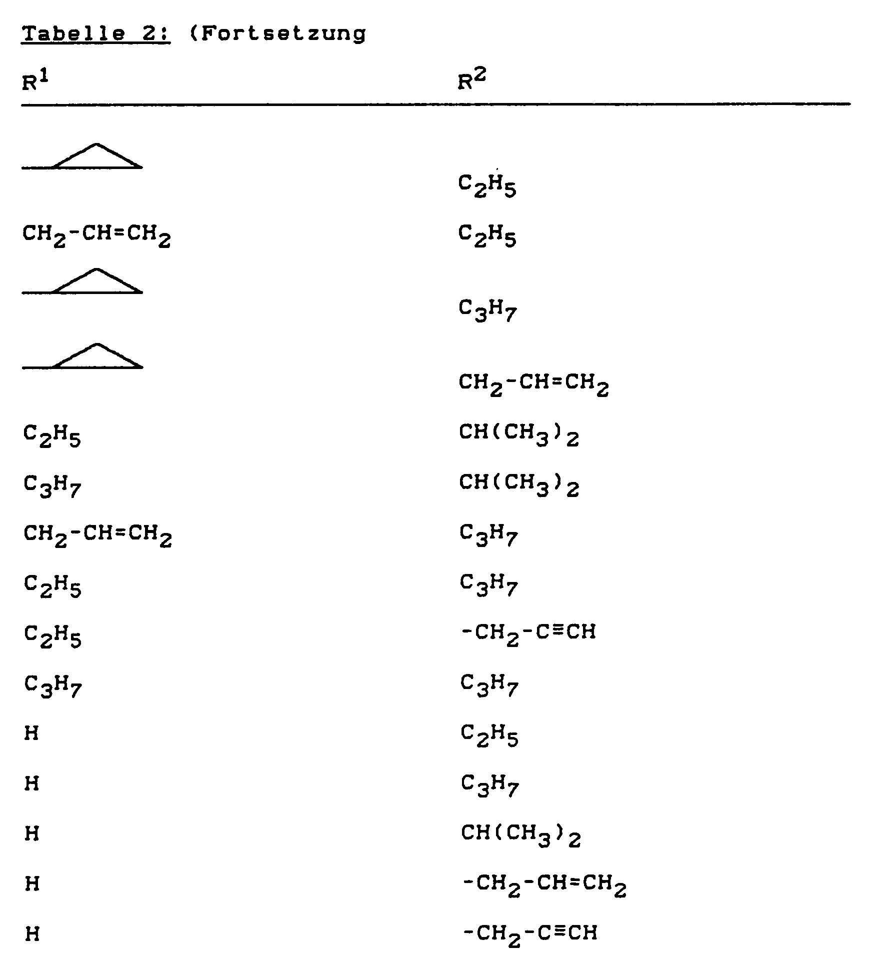

Die Ausgangsstoffe der Formel (II) sind mit Ausnahme der Verbindungen 4-Hydroxy-5-methoxy- und 4-Hydroxy-5-ethoxy-2,4-dihydro-3H-1,2,4-triazol-3-on [vgl. Arch. Pharm. (Weinheim) Bd. 301, S. 827-829 (1968)] noch nicht aus der Literatur bekannt und sind als neue Stoffe auch Gegenstand der vorliegenden Patentanmeldung.

Man erhält die Verbindungen der Formel (II), wenn man 4-Hydroxy-semicarbazid der Formel (VII)

H₂N-NH-CO-NH-OH (VII)

mit Orthokohlensäureestern der allgemeinen Formel (VIII)

C(OR²)₄ (VIII)

in welcher
- R²: die oben angegebene Bedeutung hat,
gegebenenfalls in Gegenwart eines Verdünnungsmittels, wie z.B. Methanol, Ethanol oder Propanol, bei Temperaturen zwischen 0°C und 150°C umsetzt und gegebenenfalls die hierbei gebildeten Verbindungen der allgemeinen Formel (IIa) 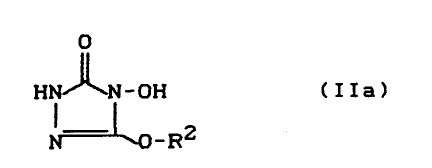 in welcher
- R²: die oben angegebene Bedeutung hat,
mit Alkylierungsmitteln der allgemeinen Formel (IX)

R¹-X (IX)

in welcher
- X: für Chlor, Brom, Jod, Methylsulfonyloxy, Trifluormethylsulfonyloxy, Phenylsulfonyloxy, Tolylsulfonyloxy oder eine der Gruppierungen -O-CO-OR¹ oder -O-SO₂-OR¹ steht und
- R¹: die oben angegebene Bedeutung, ausgenommen Wasserstoff, hat,
gegebenenfalls in Gegenwart eines Verdünnungsmittels, wie z.B. Methanol, Ethanol oder Propanol, und gegebenenfalls in Gegenwart eines Säurebindemittels, wie z.B. Natriumhydroxid, Kaliumcarbonat oder Triethylamin, bei Temperaturen zwischen -30°C und +100°C umsetzt (vgl. die Herstellungsbeispiele).

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In Formel (III) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonylisocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren (a) wird im allgemeinen bei Normaldruck durchgeführt.

Zur Durchführung des erfindungsgemäßen Verfahrens (a) setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt und das Produkt durch Absaugen isoliert. In einer anderen Aufarbeitungsvariante wird eingeengt und das im Rückstand verbleibende Rohprodukt mit einem geeigneten Lösungsmittel, wie z. B. Diethylether, zur Kristallisation gebracht. Das hierbei kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinon-Derivate sind durch die Formel (IV) allgemein definiert.

In Formel (IV) haben R¹ und R² vorzugsweise diejenigen Bedeutungen, die bereits oben im Zusammenhang mit der Beschreibung der erfindungsgemäßen Verbindungen der Formel (I) vorzugsweise für R¹ und R² angegeben wurden und
- Z: steht vorzugsweise für Methoxy oder Phenoxy.

Mögliche Beispiele für die Ausgangsstoffe der Formel (IV) sind die aus den in Tabelle 2 aufgeführten Verbindungen der Formel (II) und Phosgen, Chlorameisensäuremethylester, Chlorameisensäure-benzylester, Chlorameisensäurephenylester oder Diphenylcarbonat herzustellenden Verbindungen der Formel (IV).

Die Ausgangsstoffe der Formel (IV) sind noch nicht bekannt und sind als neue Stoffe ebenfalls Gegenstand der vorliegenden Erfindung.

Man erhält die neuen Triazolinon-Derivate der Formel (IV), wenn man Triazolinone der allgemeinen Formel (II) 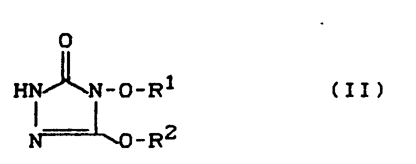 in welcher
- R¹ und R²: die oben angegebenen Bedeutungen haben,
mit Kohlensäurederivaten der allgemeinen Formel (X)

Z-CO-Z¹ (X)

in welcher
- Z: die oben angegebene Bedeutung hat und
- Z¹: für eine Abgangsgruppe wie Chlor, Methoxy, Benzyloxy oder Phenoxy steht,
gegebenenfalls in Gegenwart eines Verdünnungsmittels, wie z.B. Tetrahydrofuran, und gegebenenfalls in Gegenwart eines Säureakzeptors, wie z.B. Natriumhydrid oder Kalium-tert-butylat, bei Temperaturen zwischen -20°C und +100°C umsetzt.

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert.

In Formel (V) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (V) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-benzolsulfonsäureamid, 2-Fluor-, 2-Chlor-, 2-Difluormethoxy-, 2-Trifluormethoxy-, 2-Methoxycarbonyl- und 2-Ethoxycarbonyl-phenylmethansulfonsäureamid, 2-Methoxycarbonyl-3-thiophensulfonsäureamid, 4-Methoxycarbonyl-und 4-Ethoxycarbonyl-1-methyl-pyrazol-5-sulfonsäureamid.

Die Sulfonsäureamide der Formel (V) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht, wie sie beispielsweise oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden Triazolinone der Formel (II) sind bereits als Ausgangsstoffe für das erfindungsgemäße Verfahren (a) beschrieben worden.

Die beim erfindungsgemäßen Verfahren (c) zur Herstellung von Verbindungen der Formel (I) weiter als Ausgangsstoffe zu verwendenden Sulfonsäureamid-Derivate sind durch die Formel (VI) allgemein definiert.

In Formel (VI) haben R³ und Z vorzugsweise diejenigen Bedeutungen, die bereits oben im Zusammenhang mit der Beschreibung der erfindungsgemäßen Verbindungen der Formel (I) bzw. (IV) vorzugsweise für R³ und Z angegeben wurden.

Das erfindungsgemäße Verfahren (c) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird gegebenenfalls in Gegenwart eines Säureakzeptors durchgeführt. Es kommen hierbei die gleichen Säurebindemittel in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Zur Überführung in Salze werden die Verbindungen der Formel (I) mit geeigneten Salzbildnern, wie z.B. Natrium- oder Kalium-hydroxid, -methylat oder -ethylat, Ammoniak, Isopropylamin, Dibutylamin oder Triethylamin, in geeigneten Verdünnungsmitteln, wie z.B. Wasser, Methanol oder Ethanol, verrührt. Die Salze können - dann gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage; Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsaure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenflache, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

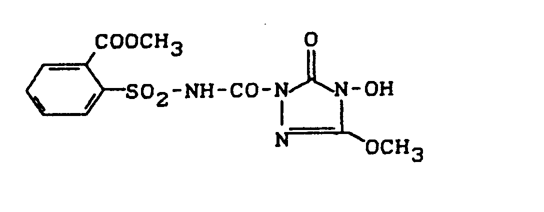

(Verfahren (a))

Eine Mischung aus 1,31 g (10 mMol) 4-Hydroxy-5-methoxy-2,4-dihydro-3H-1,2,4-triazol-3-on, 2,89 g (12 mMol) 2-Methoxycarbonyl-phenylsulfonylisocyanat und 40 ml Acetonitril wird 5 Stunden bei 20°C gerührt und anschließend im Wasserstrahlvakuum eingeengt. Der Rückstand wird mit Diethylether verrührt, das kristallin angefallene Produkt durch Absaugen isoliert und aus Isopropanol umkristallisiert.

Man erhält 1,22 g (33% der Theorie) 4-Hydroxy-5-methoxy-1-(2-methoxycarbonyl-phenylsulfonylaminocarbonyl)-2,4-dihydro-3H-1,2,4-triazol-3-on vom Schmelzpunkt 180°C.

Analog Beispiel 1 und entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

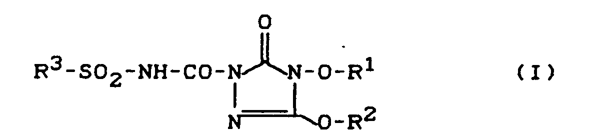

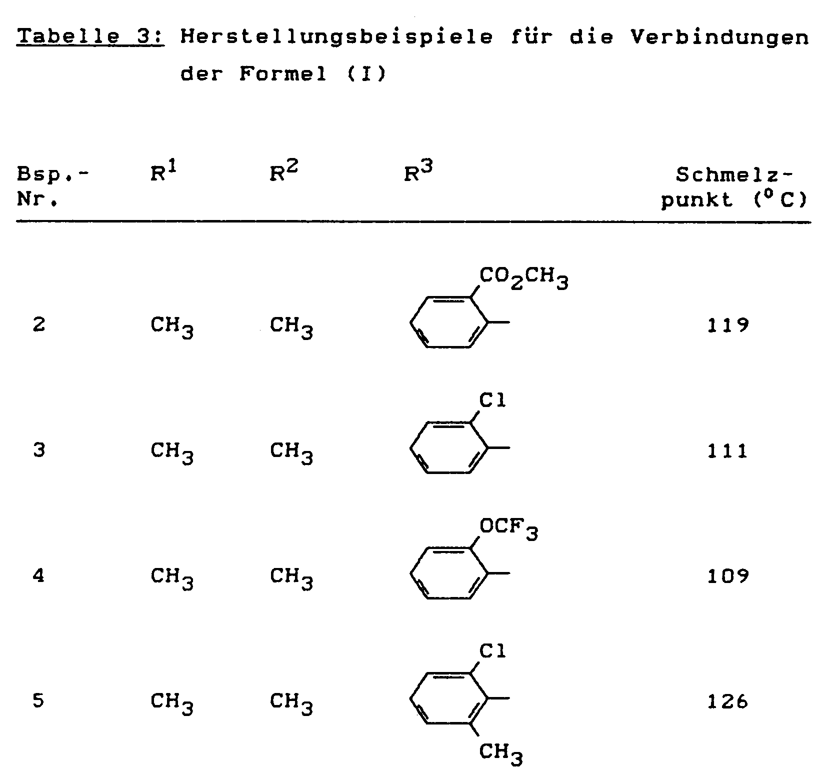

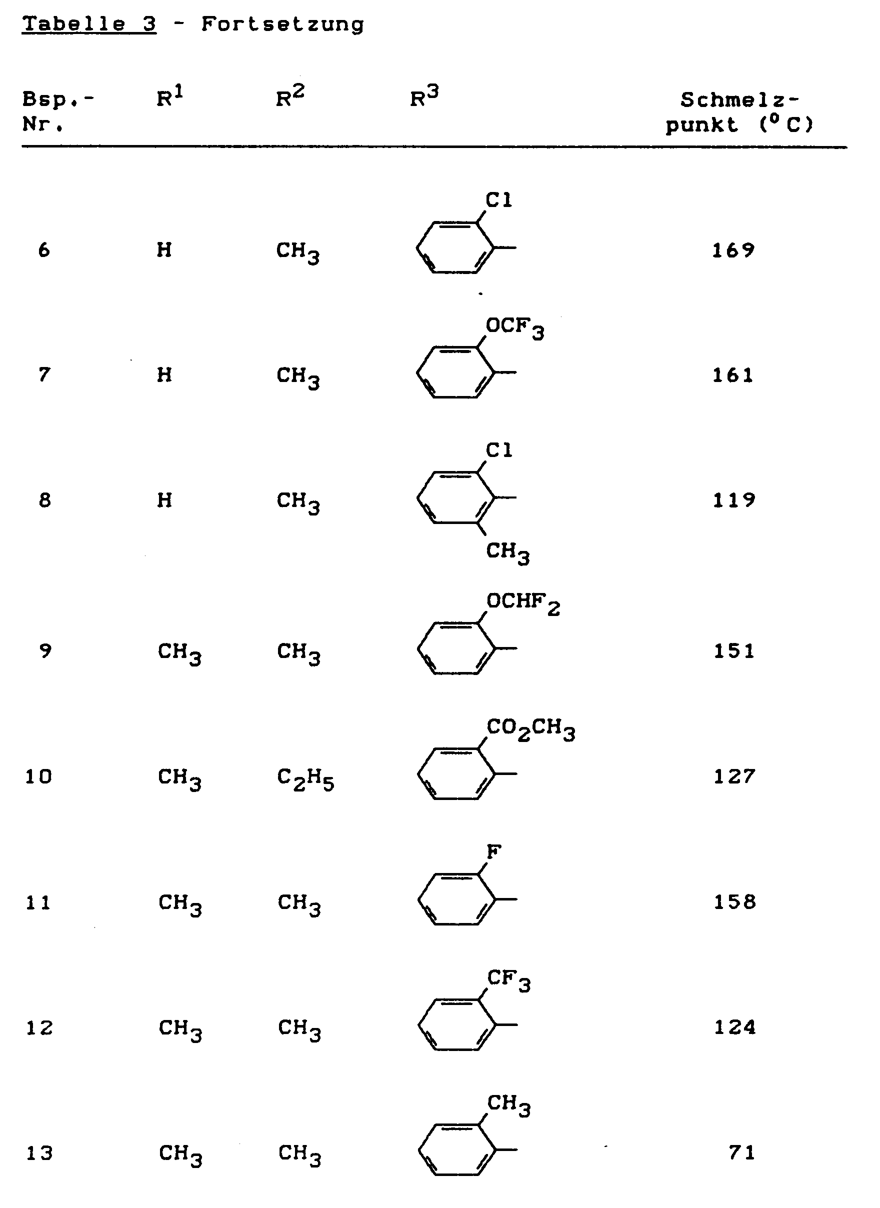

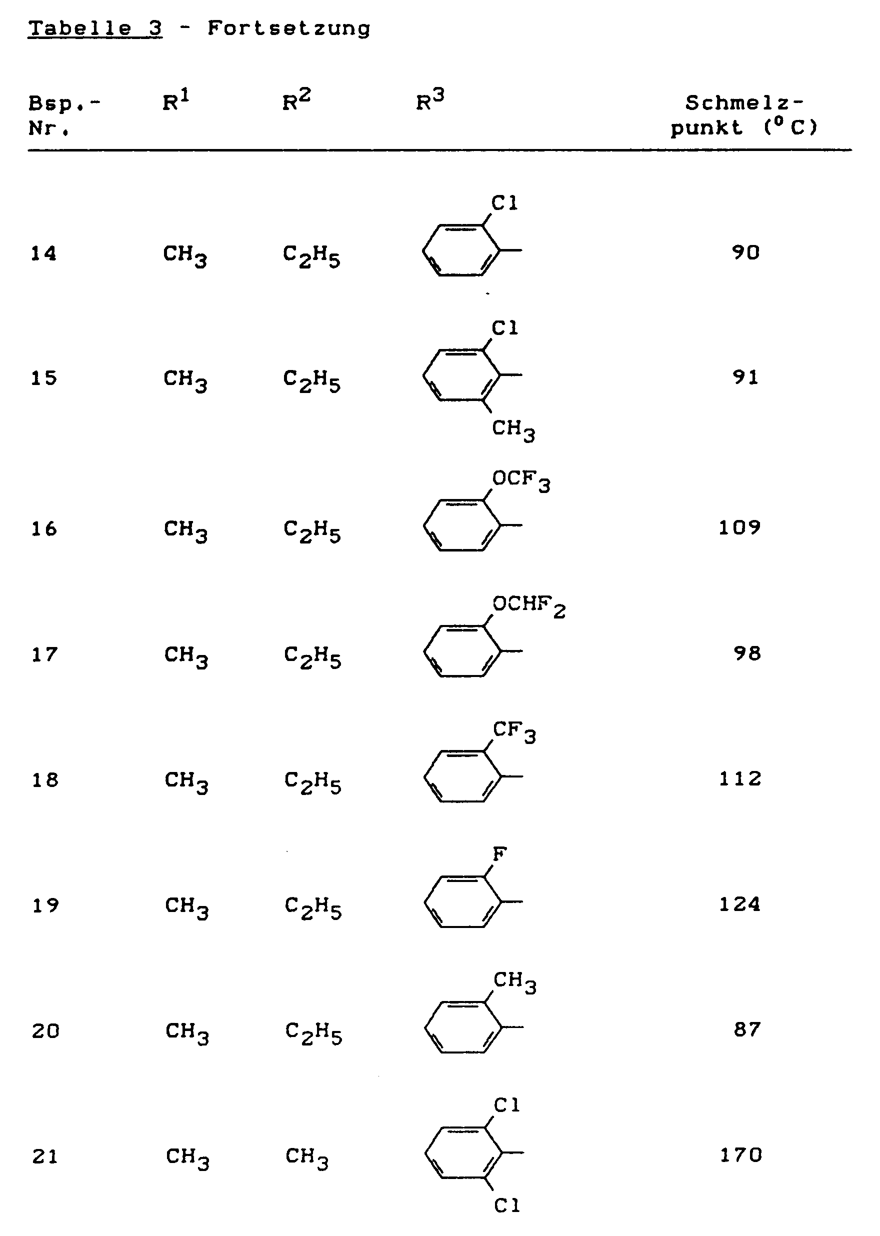

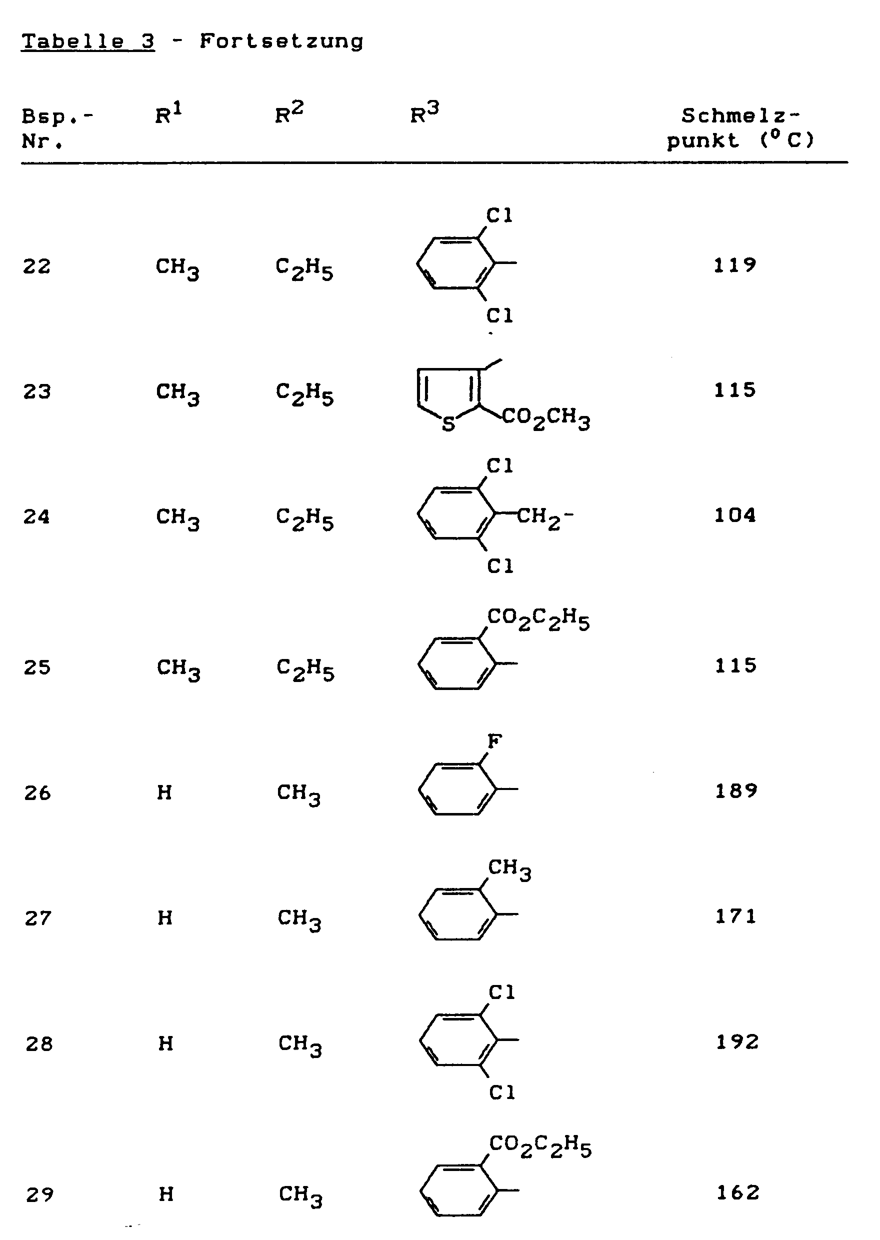

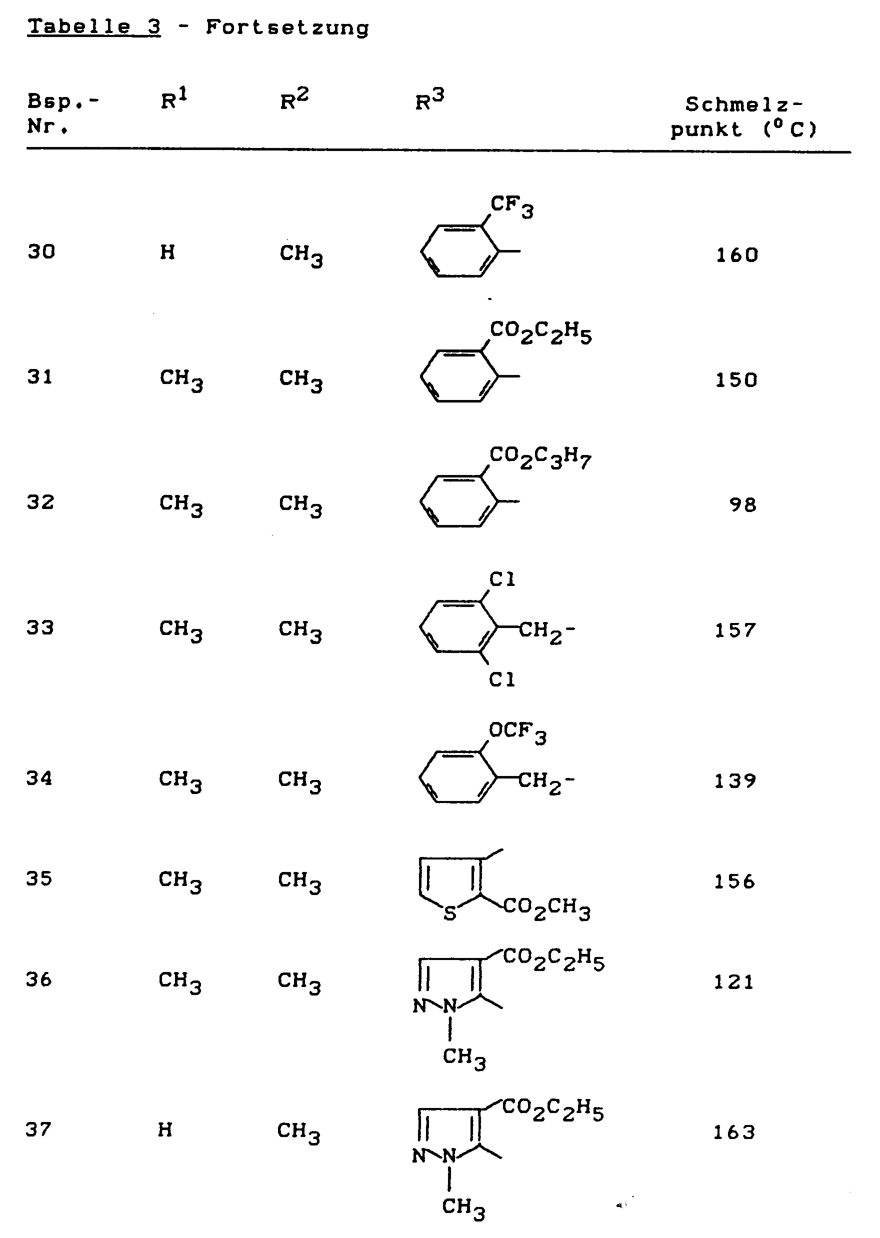

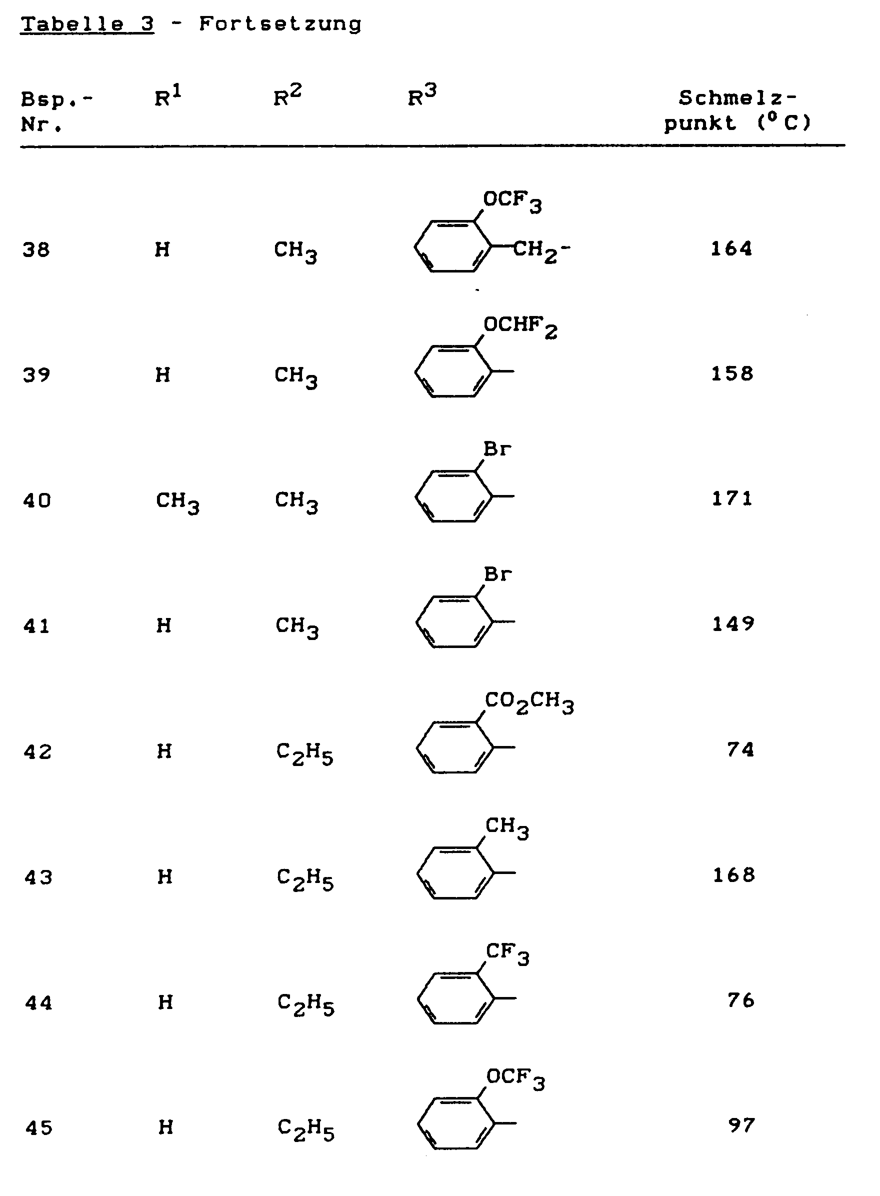

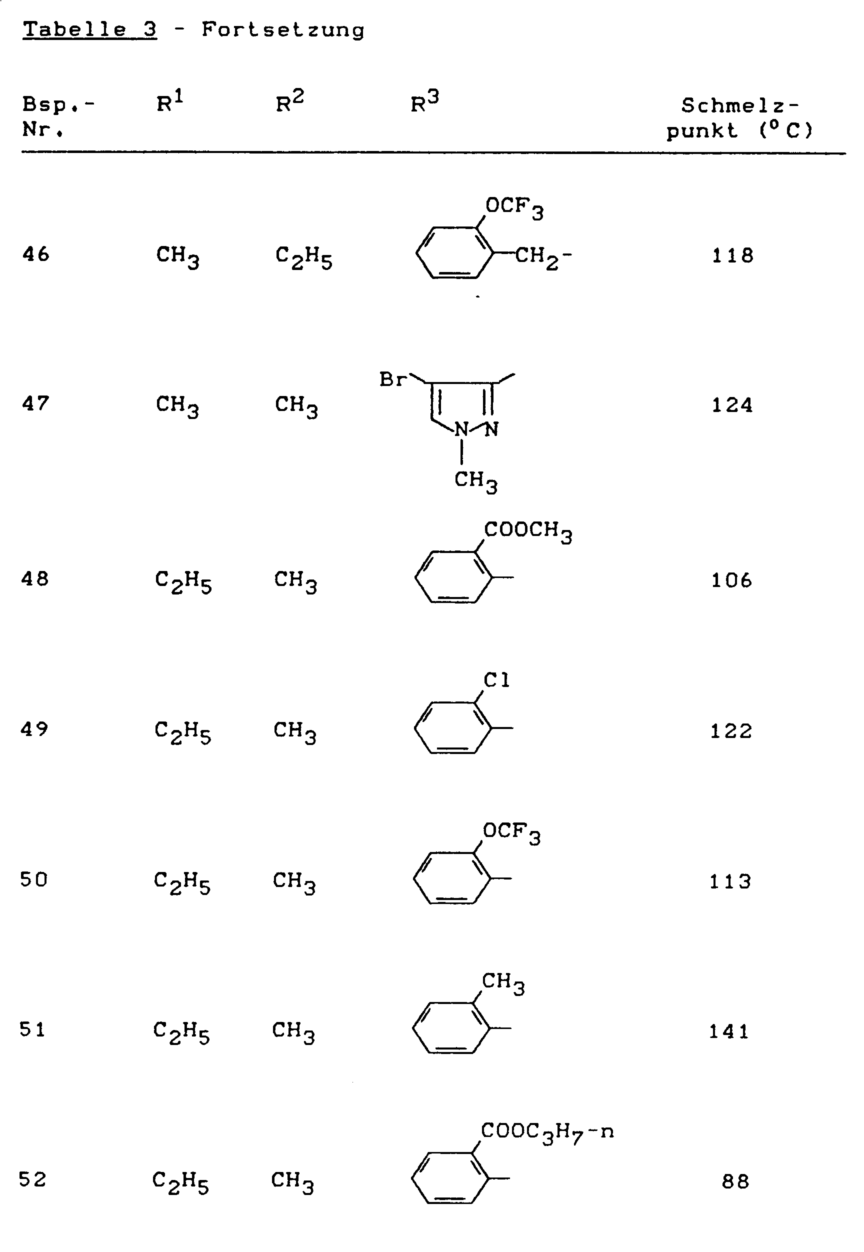

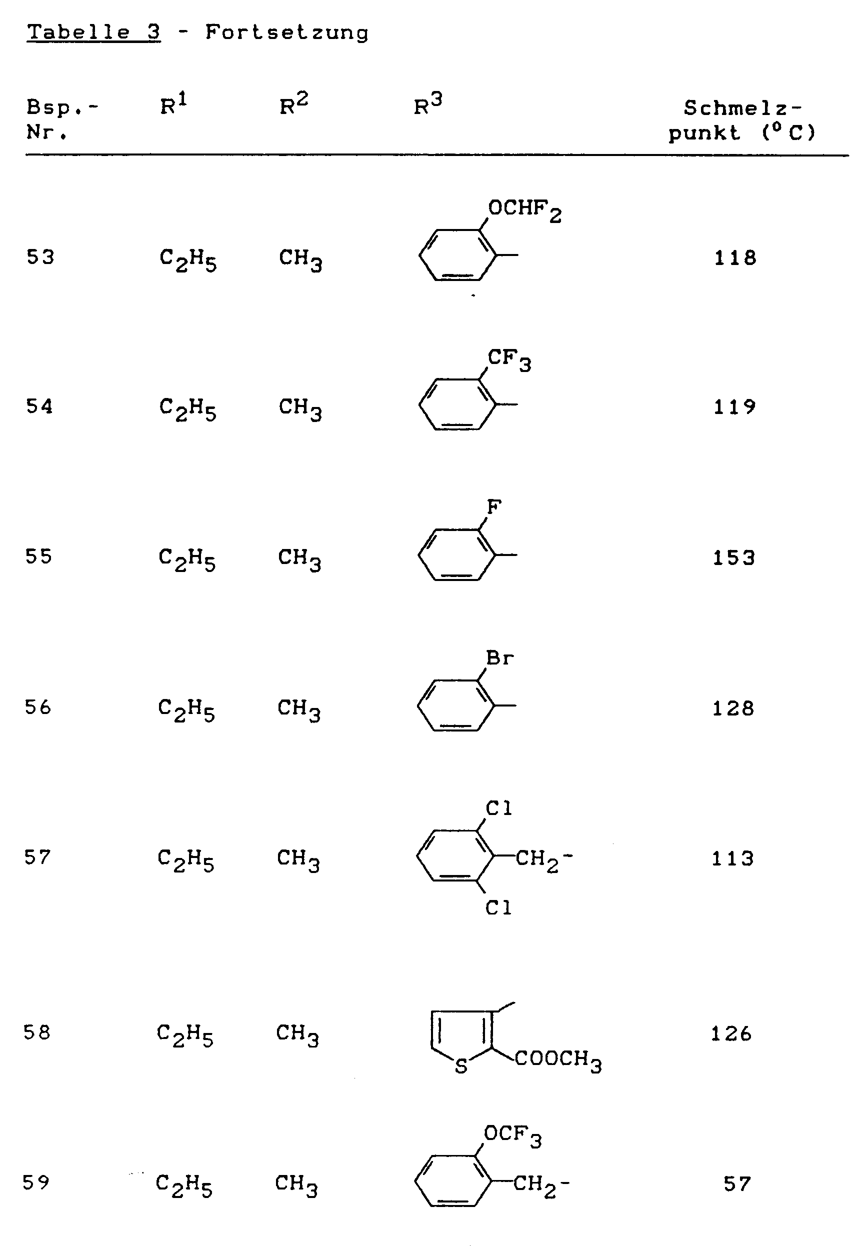

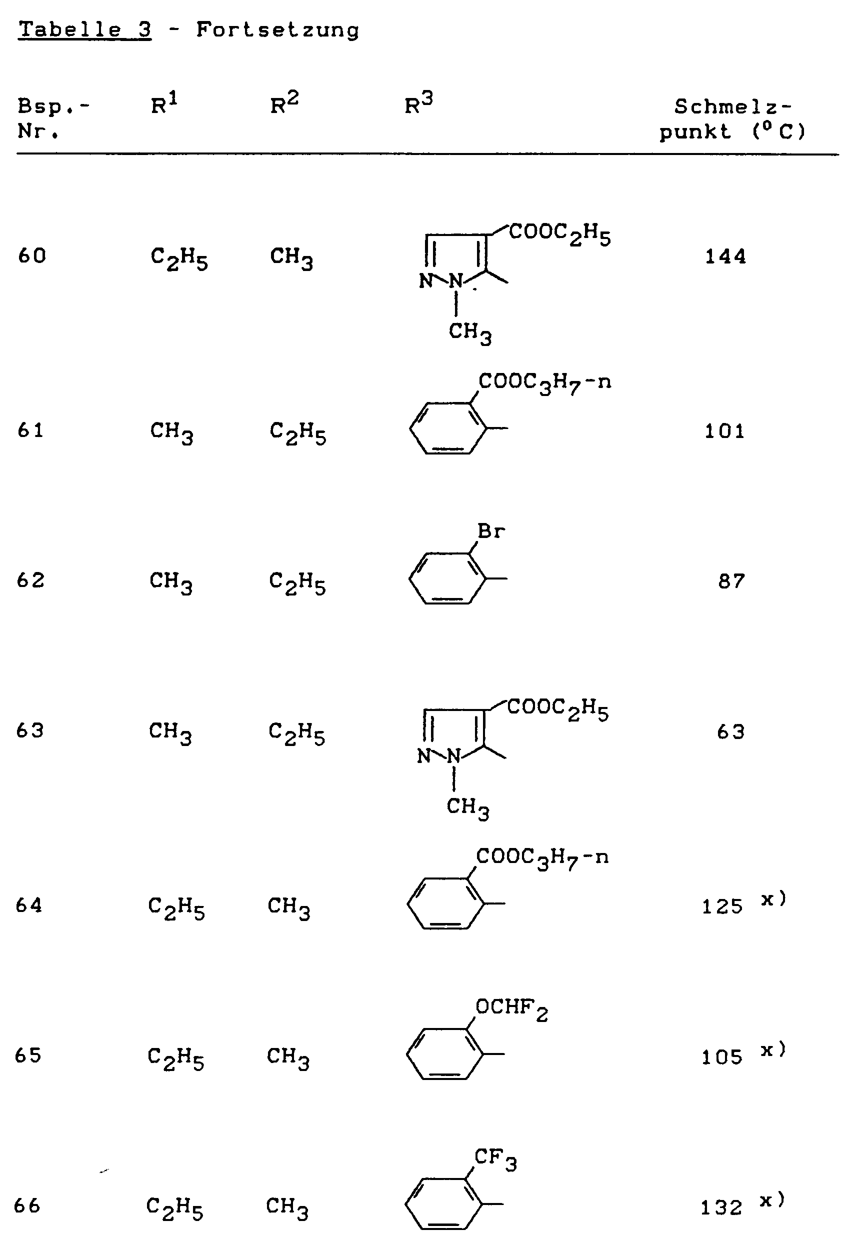

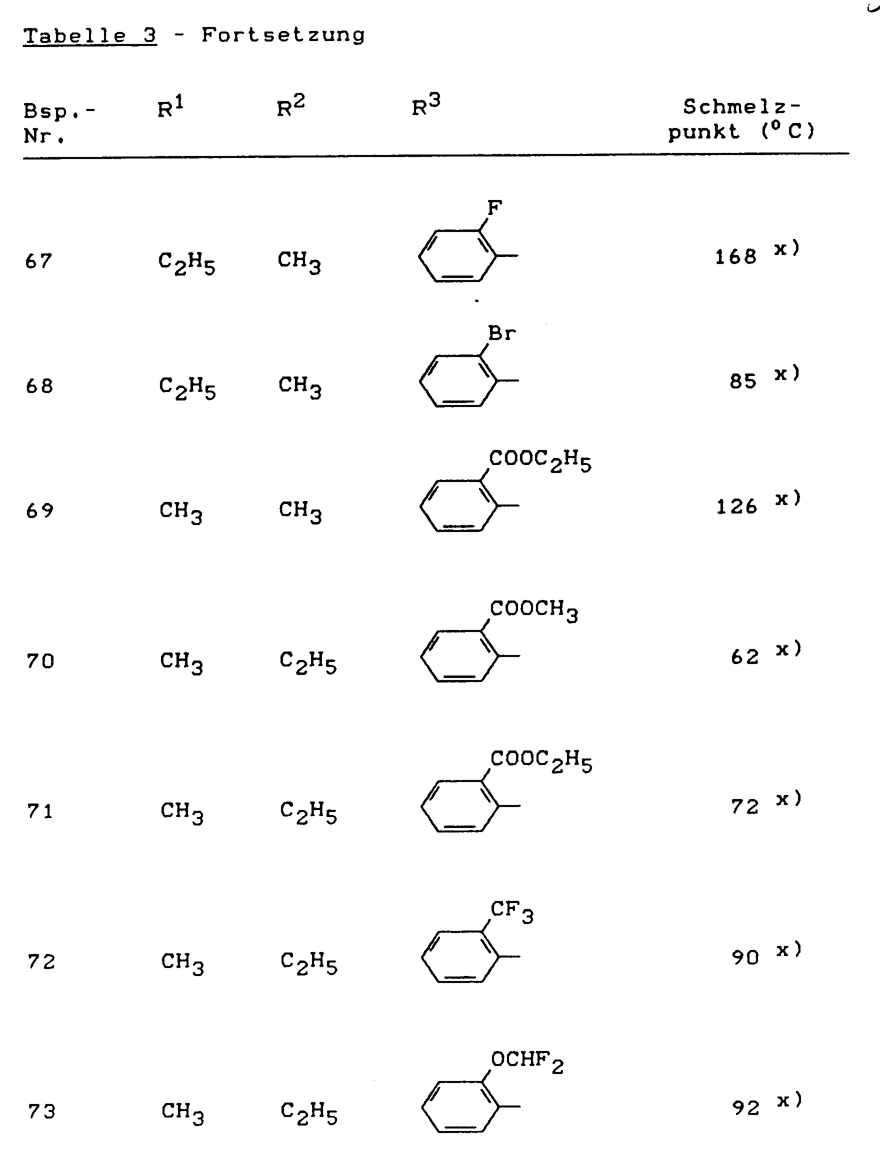

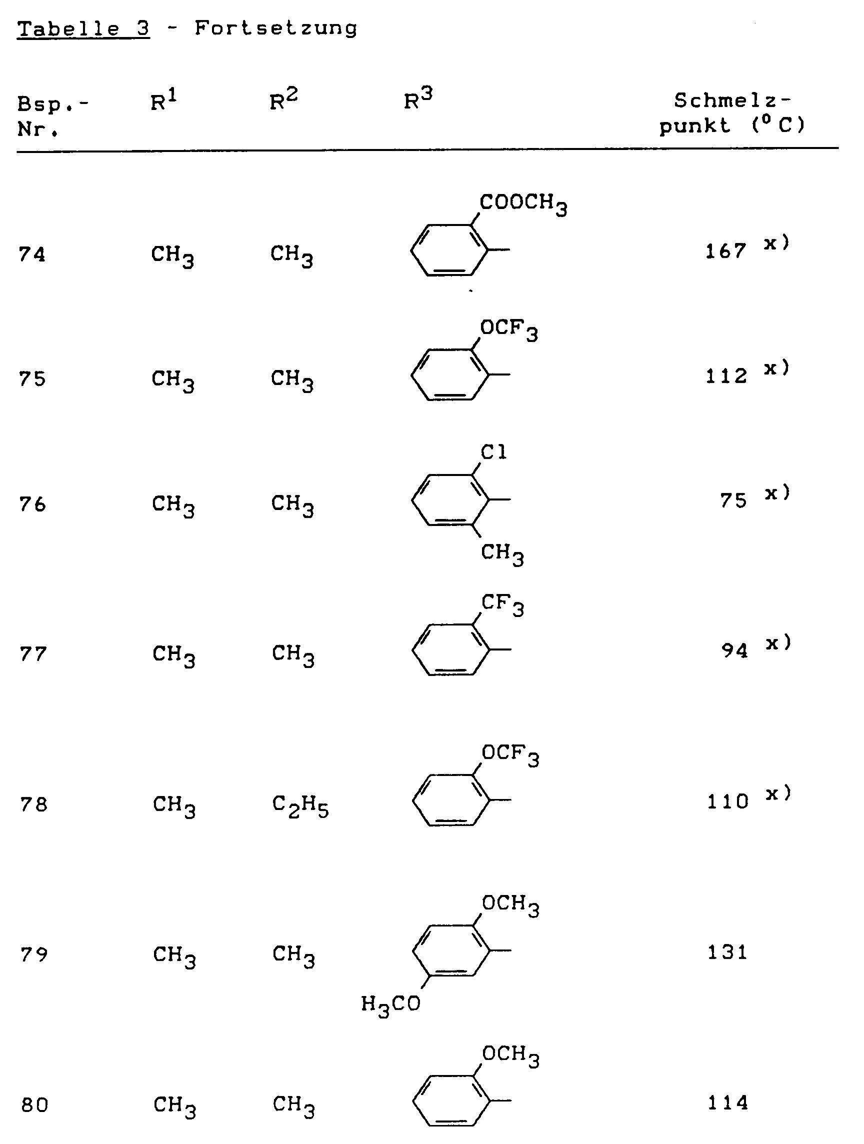

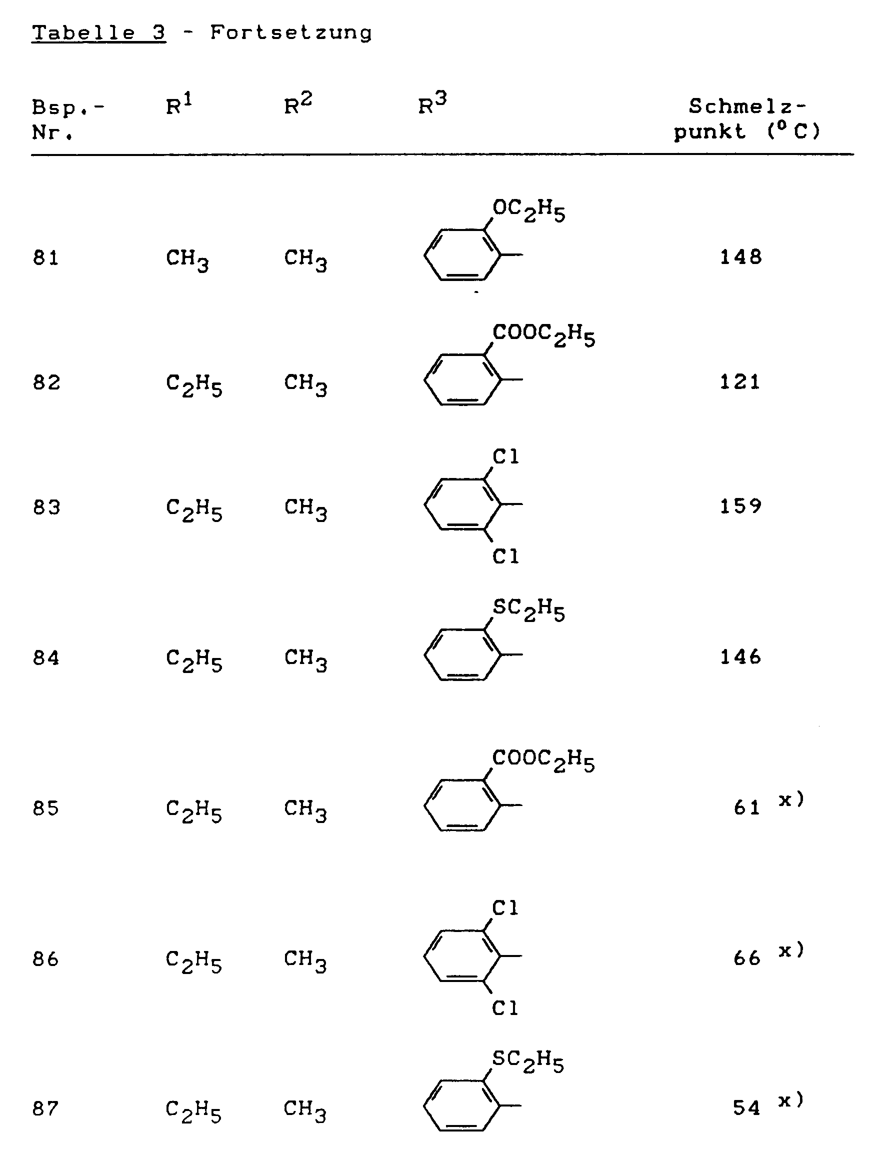

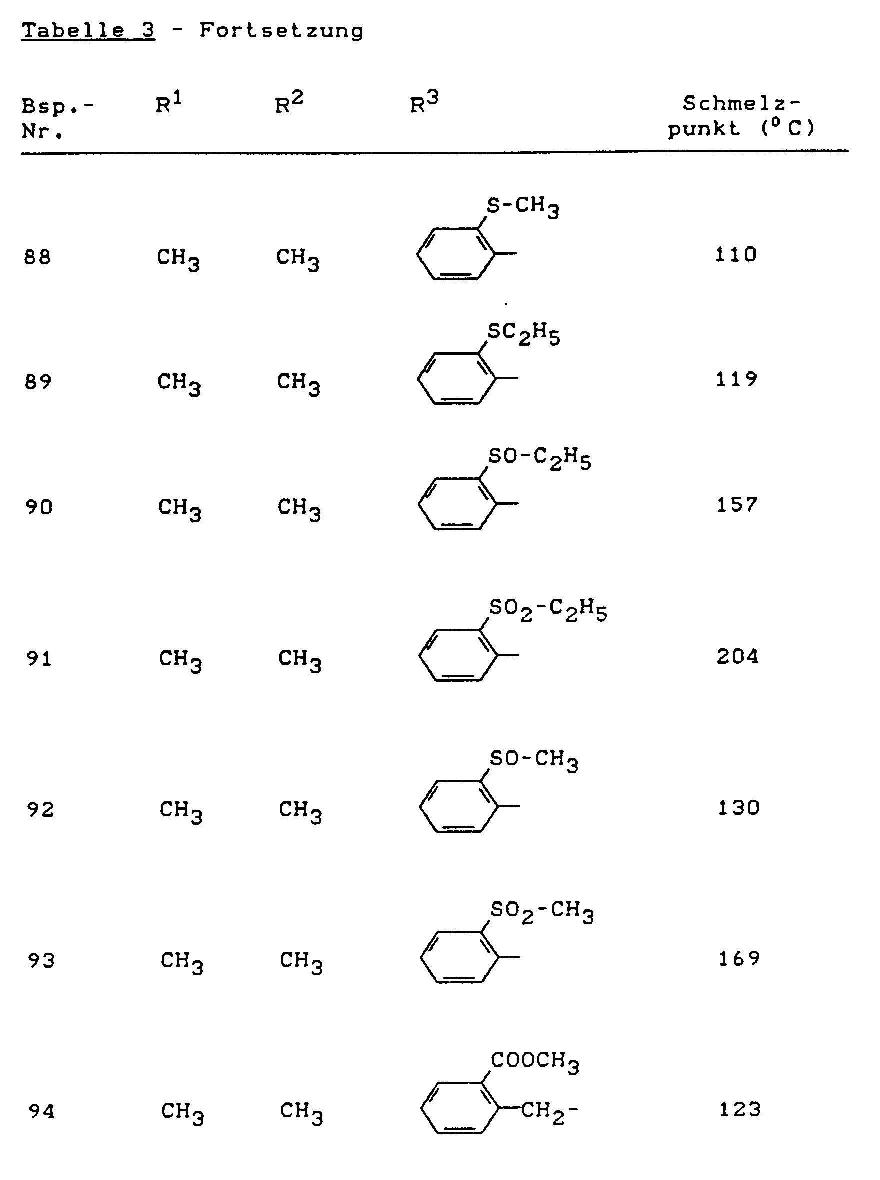

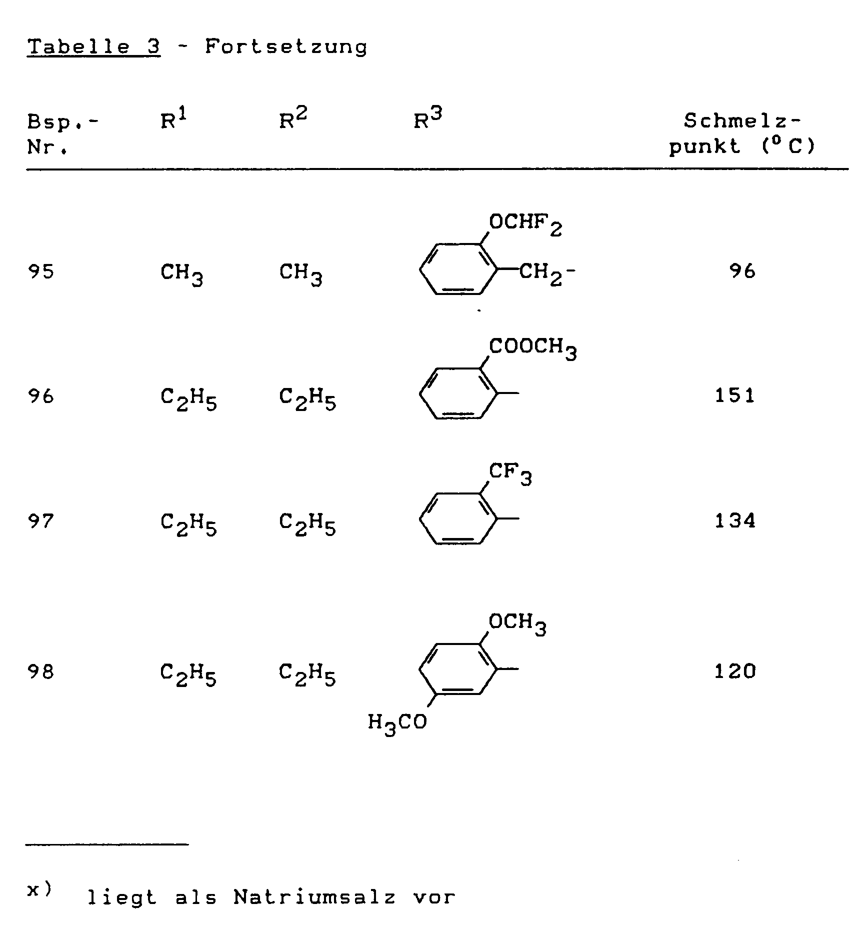

### Ausgangsstoffe der Formel (II):

### Beispiel (II-1)

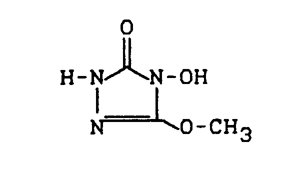

Ein Gemisch aus 42 g (0,46 Mol) 4-Hydroxysemicarbazid [R. Ohme, H. Preuschhof, J. Prakt. Chem. 313 (1971), S. 626], 73,3 g (0,54 Mol) Tetramethylorthocarbonat und 300 ml Methanol wird 18 Stunden zum Rückfluß erhitzt. Nach dem Erkalten der Reaktionslösung destilliert man 250 ml des eingesetzten Lösungsmittels im Wasserstrahlvakuum ab. Das im Rückstand kristallin anfallende farblose Produkt wird durch Absaugen isoliert.

Man erhält 22 g (36% der Theorie) 4-Hydroxy-5-methoxy-2,4-dihydro-3H-1,2,4-triazol-3-on vom Schmelzpunkt 211°C (exotherme Zersetzung); [vgl. Arch. Pharm. Bd. 301, S. 829 (1968)].

### Beispiel (II-2)

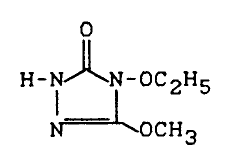

3,7 g (28 mMol) 4-Hydroxy-5-methoxy-2,4-dihydro-3H-1,2,4-triazol-3-on werden in 30 ml Ethanol suspendiert und mit 1,12 g (28 mMol) Natriumhydroxid, gelöst in 5 ml Wasser versetzt. Nach 2 Stunden Nachrührzeit bei Raumtemperatur (20°C) gibt man 4.3 g (28 mMol) Diethylsulfat hinzu und rührt weitere 18h bei Raumtemperatur. Das Reaktionsgemisch wird mit verdünnter Salzsäure angesäuert (pH = 3-4), filtriert und im Wasserstrahlvakuum eingeengt. Man verrührt den Rückstand mit 50 ml Methanol/Diethylether (1:4) und trennt die unlöslichen Bestandteile durch Filtration ab. Das durch Einengen des Filtrats anfallende Rohprodukt wird durch Umkristallisation aus Isopropanol gereinigt.

Man erhält 1,8 g (40% der Theorie) 4-Ethoxy-5-methoxy-2,4-dihydro-3H-1,2,4-triazol-3-on als farblosen Feststoff vom Schmelzpunkt 57°C.

Analog zu den Beispielen (II-1) und (II-2) können beispielsweise auch die in der nachstehenden Tabelle 4 aufgeführten Verbindungen der Formel (II) hergestellt werden.

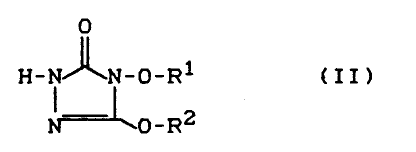

**Tabelle 4**

| Beispiele für die Ausgangsstoffe der Formel (II) | | | |
|---|---|---|---|
| Bsp.-Nr. | R¹ | R² | Schmelzpunkt (°C) |
| II-3 | CH₃ | CH₃ | 147 |
| II-4 | H | C₂H₅ | 145 |
| II-5 | CH₃ | C₂H₅ | 118 |

### Anwendungsbeispiele:

In den Anwendungsbeispielen wird die folgende Verbindung (A) als Vergleichssubstanz herangezogen:

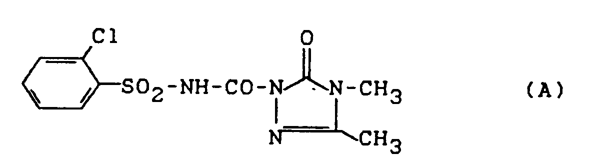

2-(2-Chlor-phenylsulfonylaminocarbonyl)-4,5-dimethyl-2,4-dihydro-3H-1,2,4-triazol-3-on (bekannt aus EP-A 341489).

### Beispiel A

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:
- 0 %: = keine Wirkung (wie unbehandelte Kontrolle)
- 100 %: = totale Vernichtung

Eine deutliche Überlegenheit in der Wirksamkeit gegenüber dem Stand der Technik zeigen in diesem Test z.B. die Verbindungen gemäß folgender Herstellungsbeispiele: 5, 12 und 13.

### Beispiel B

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 %: = keine Wirkung (wie unbehandelte Kontrolle)
- 100 %: = totale Vernichtung

Eine deutliche Überlegenheit in der Wirksamkeit gegenüber dem Stand der Technik zeigen in diesem Test z.B. die Verbindungen gemäß folgender Herstellungsbeispiele: 2, 4 und 20.

## Patentansprüche (Patentansprüche für folgende(n) Vertragsstaat(en): BE, CH, DE, FR, GB, IT, LI, NL)

1. Sulfonylaminocarbonyltriazolinone mit zwei über Sauerstoff gebundenen Substituenten der allgemeinen Formel (I), 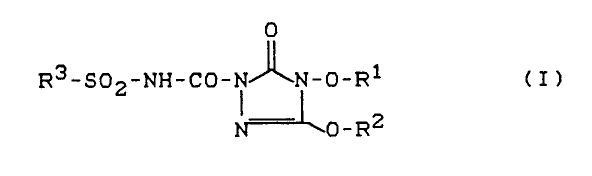 in welcher
R¹ für Wasserstoff, für gegebenenfalls durch Fluor, Chlor, Cyano oder C₁-C₄-Alkoxy substituiertes C₁-C₆-Alkyl, für gegebenenfalls durch Fluor, Chlor und/oder Brom substituiertes C₃-C₆-Alkenyl oder für gegebenenfalls durch Fluor, Chlor, Brom und/oder C₁-C₄-Alkyl substituiertes C₃-C₇-Cycloalkyl steht,
R² für gegebenenfalls durch Fluor, Chlor, C₃-C₆-Cycloalkyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl oder für Cyclohexenyl steht und
R³ für die Gruppierung 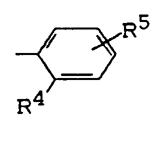 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 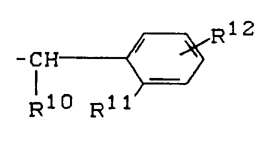 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄ Alkylsulfonyl oder Di-(C₁-C₄-alkyl)-aminosulfonyl stehen;
weiterhin
R³ für den Rest 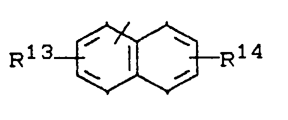 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 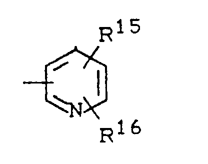 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Flu or und/oder Chlor substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsul fonyl (welche gegebenenfalls durch Fluor und/oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
R³ für den Rest 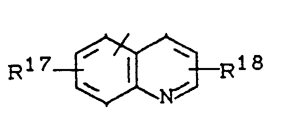 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen;
weiterhin
R³ für den Rest 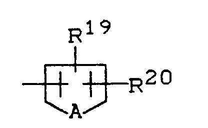 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 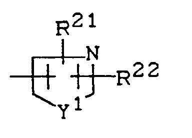 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 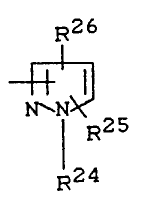 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht: 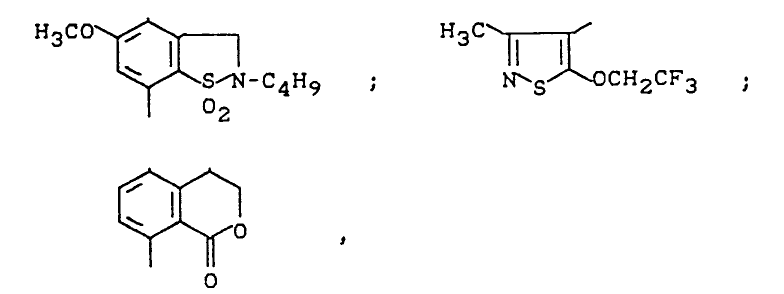
sowie Salze von Verbindungen der Formel (I).

2. Verbindungen der Formel (I) gemäß Anspruch 1, dadurch gekennzeichnet, daß darin
R¹ für Wasserstoff, für gegebenenfalls durch Fluor, Cyano, Methoxy oder Ethoxy substituiertes C₁-C₄-Alkyl, für Allyl oder für C₃-C₆-Cycloalkyl steht,
R² für gegebenenfalls durch Fluor und/oder Chlor, Methoxy oder Ethoxy substituiertes C₁-C₄-Alkyl, für gegebenenfalls durch Fluor und/oder Chlor substituiertes C₃-C₄-Alkenyl oder für C₃-C₆-Cycloalkyl steht, und
R³ für die Gruppierung 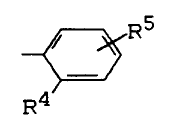 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkyl-sulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 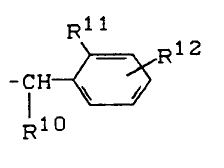 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 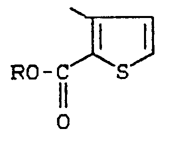 steht, worin R für C₁-C₄-Alkyl steht, oder
für den Rest 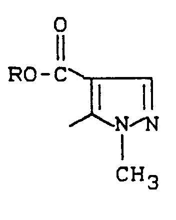 steht,
worin R für C₁-C₄-Alkyl steht.

3. Verfahren zur Herstellung von Sulfonylaminocarbonyltriazolinonen mit zwei über Sauerstoff gebundenen Substituenten der allgemeinen Formel (I) gemäß Anspruch 1 sowie von Salzen von Verbindungen der Formel (I),
dadurch gekennzeichnet, daß man
(a) Triazolinone der allgemeinen Formel (II) 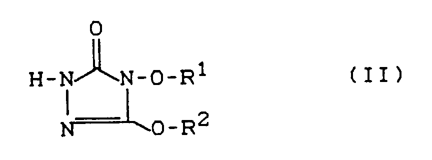 in welcher
R¹ und R² die in Anspruch 1 angegebenen Bedeutungen
haben, mit Sulfonylisocyanaten der allgemeinen Formel (III)
R³-SO₂-N=C=O (III)
in welcher
R³ die in Anspruch 1 angegebene Bedeutung hat,
gegebenenfalls in Gegenwart eines Verdünnungsmittels umsetzt, oder daß man
(b) Triazolinon-Derivate der allgemeinen Formel (IV) 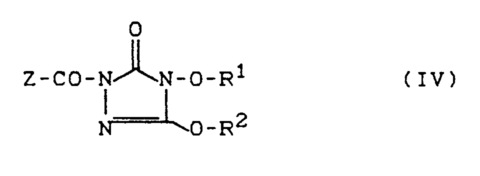 in welcher
R¹ und R² die oben angegebenen Bedeutungen haben und
Z für Chlor, C₁-C₄-Alkoxy, Benzyloxy oder Phenoxy steht,
mit Sulfonsäureamiden der allgemeinen Formel (V)
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt, oder daß man
(c) Triazolinone der allgemeinen Formel (II) 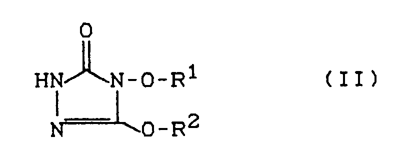 in welcher
R¹ und R² die oben angegebenen Bedeutungen haben,
mit Sulfonsäureamid-Derivaten der allgemeinen Formel (VI)
R³-SO₂-NH-CO-Z (VI)
in welcher
R³ die oben angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt
und gegebenenfalls aus den nach Verfahren (a), (b) oder (c) hergestellten Verbindungen der Formel (I) nach üblichen Methoden Salze erzeugt.

4. Herbizide Mittel, gekennzeichnet durch einen Gehalt an mindestens einem Sulfonylaminocarbonyltriazolinon der Formel (I) gemäß Anspruch 1.

5. Verwendung von Sulfonylaminocarbonyltriazolinonen der allgemeinen Formel (I) gemäß Anspruch 1 zur Bekämpfung von unerwünschtem Pflanzenwachstum.

6. Verfahren zur Bekämpfung von Unkräutern, dadurch gekennzeichnet, daß man Sulfonylaminocarbonyltriazolinone der allgemeinen Formel (I) gemäß Anspruch 1 auf die Unkräuter oder ihren Lebensraum einwirken läßt.

7. Verfahren zur Herstellung von herbiziden Mitteln, dadurch gekennzeichnet, daß man Sulfonylaminocarbonyltriazolinone der allgemeinen Formel (I) gemäß Anspruch 1 mit Streckmitteln und/oder oberflächenaktiven Mitteln vermischt.

8. Triazolinone der allgemeinen Formel (II) 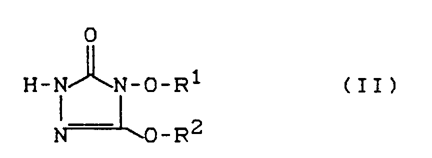
R¹ für Wasserstoff, für gegebenenfalls durch Fluor, Chlor, Cyano oder C₁-C₄-Alkoxy substituiertes C₁-C₆-Alkyl, für gegebenenfalls durch Fluor, Chlor und/oder Brom substituiertes C₃-C₆-Alkenyl oder für gegebenenfalls durch Fluor, Chlor, Brom und/oder C₁-C₄-Alkyl substituiertes C₃-C₇-Cycloalkyl steht und
R² für gegebenenfalls durch Fluor, Chlor, C₃-C₆-Cycloalkyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl oder für Cyclohexenyl steht,
ausgenommen die Verbindungen 4-Hydroxy-5-methoxy-2,4-dihydro-3H-1,2,4-triazol-3-on und 4-Hydroxy-5-ethoxy-2,4-dihydro-3H-1,2,4-triazol-3-on.

9. Triazolinon-Derivate der allgemeinen Formel (IV) 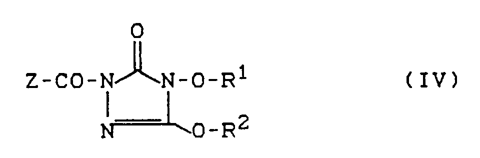 in welcher
R¹ für Wasserstoff, für gegebenenfalls durch Fluor, Chlor, Cyano oder C₁-C₄-Alkoxy substituiertes C₁-C₆-Alkyl, für gegebenenfalls durch Fluor, Chlor und/oder Brom substituiertes C₃-C₆-Alkenyl oder für gegebenenfalls durch Fluor, Chlor, Brom und/oder C₁-C₄-Alkyl substituiertes C₃-C₇-Cycloalkyl steht,
R² für gegebenenfalls durch Fluor, Chlor, C₃-C₆-Cycloalkyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl oder für Cyclohexenyl steht und
Z für Chlor, C₁-C₄-Alkoxy, Benzyloxy oder Phenoxy steht.

## Patentansprüche (Patentansprüche für folgende(n) Vertragsstaat(en): ES)

1. Verfahren zur Herstellung von Sulfonylaminocarbonyltriazolinonen mit zwei über Sauerstoff gebundenen Substituenten der allgemeinen Formel (I) 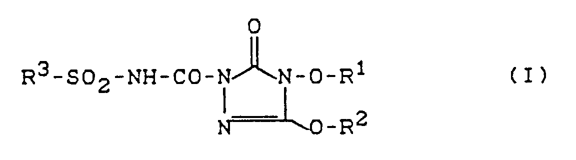 in welcher
R¹ für Wasserstoff, für gegebenenfalls durch Fluor, Chlor, Cyano oder C₁-C₄-Alkoxy substituiertes C₁-C₆-Alkyl, für gegebenenfalls durch Fluor, Chlor und/oder Brom substituiertes C₃-C₆-Alkenyl oder für gegebenenfalls durch Fluor, Chlor, Brom und/oder C₁-C₄-Alkyl substituiertes C₃-C₇-Cycloalkyl steht,
R² für gegebenenfalls durch Fluor, Chlor, C₃-C₆-Cycloalkyl oder C₁-C₄-Alkoxy substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl oder für Cyclohexenyl steht und
R³ für die Gruppierung 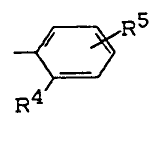 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₂-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 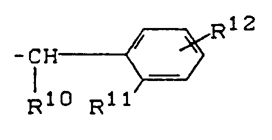 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Carboxy, C₁-C₄-Alkoxy-carbonyl, Dimethylaminocarbonyl, C₁-C₄ Alkylsulfonyl oder Di-(C₁-C₄-alkyl)-aminosulfonyl stehen;
weiterhin
R³ für den Rest 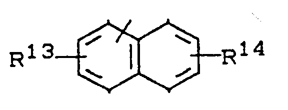 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 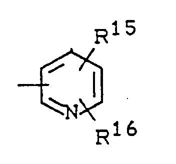 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Flu or und/oder Chlor substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsul fonyl (welche gegebenenfalls durch Fluor und/oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
R³ für den Rest 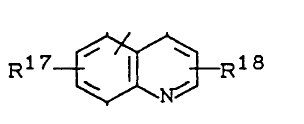 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen;
weiterhin
R³ für den Rest 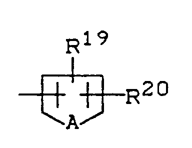 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 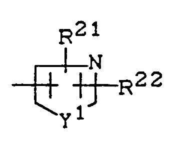 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 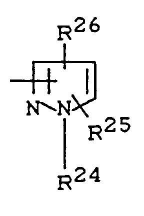 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht: 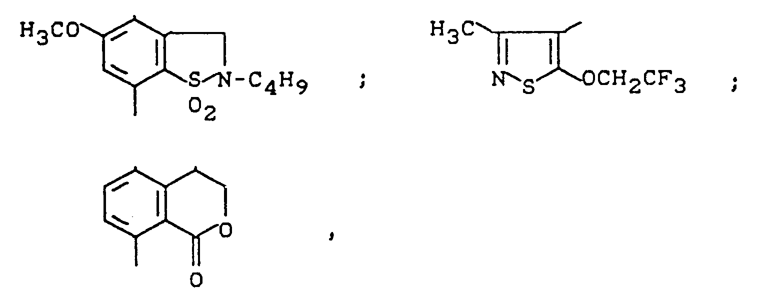
sowie von Salzen von Verbindungen der Formel (I),
dadurch gekennzeichnet, daß man
(a) Triazolinone der allgemeinen Formel (II) 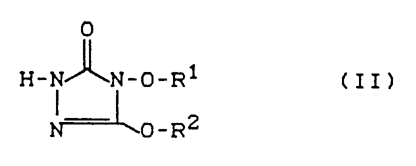 in welcher
R¹ und R² die oben angegebenen Bedeutungen haben,
mit Sulfonylisocyanaten der allgemeinen Formel (III)
R³-SO₂-N=C=O (III)
in welcher
R³ die oben angegebene Bedeutung hat,
gegebenenfalls in Gegenwart eines Verdünnungsmittels umsetzt, oder daß man
(b) Triazolinon-Derivate der allgemeinen Formel (IV) 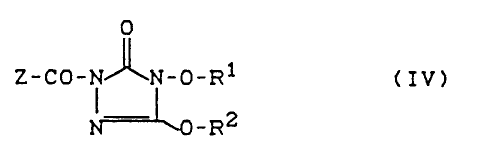 in welcher
R¹ und R² die oben angegebenen Bedeutungen haben und
Z für Chlor, C₁-C₄-Alkoxy, Benzyloxy oder Phenoxy steht,
mit Sulfonsäureamiden der allgemeinen Formel (V)
R³-SO₂-NH₂ (V)
in welcher
R³ die oben angegebene Bedeutung hat,
gegebenenfalls in Gegenwart eines Säureakzeptors und gegebenenfalls in Gegenwart eines Verdünnungsmittels umsetzt, oder daß man
(c) Triazolinone der allgemeinen Formel (II) 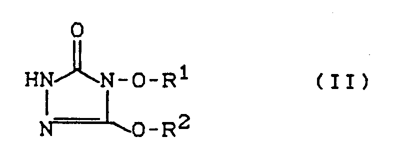 in welcher
R¹ und R² die oben angegebenen Bedeutungen haben,
mit Sulfonsäureamid-Derivaten der allgemeinen Formel (VI)
R³-SO₂-NH-CO-Z (VI)
in welcher
R³ die oben angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt
und gegebenenfalls aus den nach Verfahren (a), (b) oder (c) hergestellten Verbindungen der Formel (I) nach üblichen Methoden Salze erzeugt.

2. Herbizide Mittel, gekennzeichnet durch einen Gehalt an mindestens einem Sulfonylaminocarbonyltriazolinon der Formel (I) gemäß Anspruch 1.

3. Verwendung von Sulfonylaminocarbonyltriazolinonen der allgemeinen Formel (I) gemäß Anspruch 1 zur Bekämpfung von unerwünschtem Pflanzenwachstum.

4. Verfahren zur Bekämpfung von Unkräutern, dadurch gekennzeichnet, daß man Sulfonylaminocarbonyltriazolinone der allgemeinen Formel (I) gemäß Anspruch 1 auf die Unkräuter oder ihren Lebensraum einwirken läßt.

5. Verfahren zur Herstellung von herbiziden Mitteln, dadurch gekennzeichnet, daß man Sulfonylaminocarbonyltriazolinone der allgemeinen Formel (I) gemäß Anspruch 1 mit Streckmitteln und/oder oberflächenaktiven Mitteln vermischt.

## Claims (Claims for the following Contracting State(s): BE, CH, DE, FR, GB, IT, LI, NL)

1. Sulphonylaminocarbonyltriazolinones having two substituents bonded via oxygen of the general formula (I) 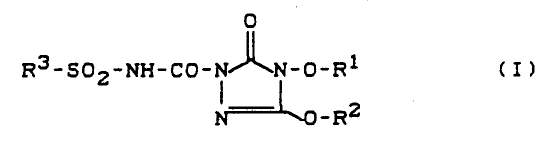 in which
R¹ represents hydrogen, or represents C₁-C₆-alkyl which is optionally substituted by fluorine, chlorine, cyano or C₁-C₄-alkoxy, or represents C₃-C₆-alkenyl which is optionally substituted by fluorine, chlorine and/or bromine, or represents C₃-C₇-cycloalkyl which is optionally substituted by fluorine, chlorine, bromine and/or C₁-C₄-alkyl,
R² represents C₁-C₆-alkyl which is optionally substituted by fluorine, chlorine, C₃-C₆-cycloalkyl or C₁-C₄-alkoxy or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl, and
R³ represents the group 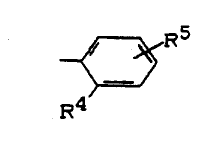 where
R⁴ and R⁵ are identical or different and represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), or represents C₂-C₆-alkenyl (which is optionally substituted by fluorine, chlorine, bromine, cyano, C₁-C₄-alkoxy-carbonyl, carboxyl or phenyl), or represents C₂-C₆-alkinyl (which is optionally substituted by fluorine, chlorine, bromine, cyano, C₁-C₄-alkoxy-carbonyl, carboxyl or phenyl), or represents C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or represents C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl), or represents C₃-C₆-alkenyloxy (which is optionally substituted by fluorine, chlorine, bromine, cyano or C₁-C₄-alkoxy-carbonyl), or represents C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio, or the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl, or the radical -NHOR⁷, where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or represents benzhydryl, or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ furthermore represent phenyl or phenoxy,
or represent C₁-C₄-alkylcarbonylamino, C₁-C₄-alkoxy-carbonylamino, C₁-C₄-alkylamino-carbonyl-amino, di-(C₁-C₄-alkyl)-amino-carbonylamino, or represent the radical -CO-R⁸, where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (each of which is optionally substituted by fluorine and/or chlorine),
R⁴ and R⁵ furthermore represent trimethylsilyl, thiazolinyl, C₁-C₄-alkylsulphonyloxy, di-(C₁-C₄-alkyl)-aminosulphonylamino or represent the radical -CH=N-R⁹, where
R⁹ represents C₁-C₆-alkyl which is optionally substituted by fluorine, chlorine, cyano, carboxyl, C₁-C₄-alkoxy, C₁-C₄-alkylthio, C₁-C₄-alkylsulphinyl or C₁-C₄-alkylsulphonyl, or represents benzyl which is optionally substituted by fluorine or chlorine, or represents C₃-C₆-alkenyl or C₃-C₆-alkinyl, each of which is optionally substituted by fluorine or chlorine, or represents phenyl which is optionally substituted by fluorine, chlorine, bromine, C₁-C₄-alkyl, C₁-C₄-alkoxy, trifluoromethyl, trifluoromethoxy or trifluoromethylthio, or represents C₁-C₆-alkoxy, C₃-C₆-alkenoxy, C₃-C₆-alkinoxy or benzyloxy, each of which is optionally substituted by fluorine and/or chlorine, or represents amino, C₁-C₄-alkylamino, di-(C₁-C₄-alkyl)-amino, phenylamino, C₁-C₄-alkyl-carbonyl-amino, C₁-C₄-alkoxy-carbonylamino, C₁-C₄-alkyl-sulphonylamino, or represents phenylsulphonylamino which is optionally substituted by fluorine, chlorine, bromine or methyl,
furthermore
R³ represents the radical 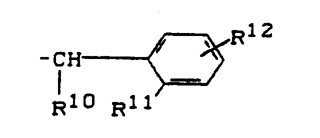 where
R¹⁰ represents hydrogen or C₁-C₄-alkyl,
R¹¹ and R¹² are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-amino sulphonyl;
furthermore
R³ represents the radical 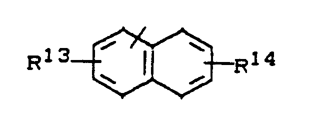 where
R¹³ and R¹⁴ are identical or different and represent hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 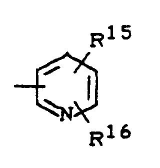 where
R¹⁵ and R¹⁶ are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent aminosulphonyl, mono-(C₁-C₄-alkyl)-aminosulphonyl, or represent di-(C₁-C₄-alkyl)-aminosulphonyl or C₁-C₄-alkoxycarbonyl or dimethylaminocarbonyl;
furthermore
R³ represents the radical 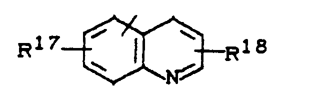 where
R¹⁷ and R¹⁸ are identical or different and represent hydrogen, fluorine, chlorine, bromine, C₁-C₄-alkyl (which is optionally substituted by fluorine and/or bromine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent di-(C₁-C₄-alkyl)-aminosulphonyl;
furthermore
R³ represents the radical 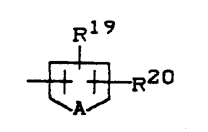 where
R¹⁹ and R²⁰ are identical or different and represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-aminosulphonyl, C₁-C₄-alkoxy-carbonyl or dimethylaminocarbonyl, and
A represents oxygen, sulphur or the group N-Z¹, where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di-(C₁-C₄-alkyl)-aminocarbonyl;
furthermore
R³ represents the radical 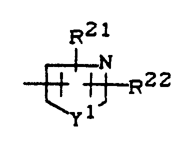 where
R²¹ and R²² are identical or different and represent hydrogen, C₁-C₄-alkyl, halogen, C₁-C₄-alkoxy-carbonyl, C₁-C₄-alkoxy or C₁-C₄-halogenoalkoxy,
Y¹ represents sulphur or the group N-R²³, where
R²³ represents hydrogen or C₁-C₄-alkyl;
furthermore
R³ represents the radical 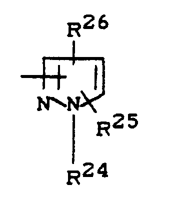 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl, and
R²⁶ represents hydrogen, halogen or C₁-C₄-alkyl;
furthermore
R³ represents one of the groups mentioned below, 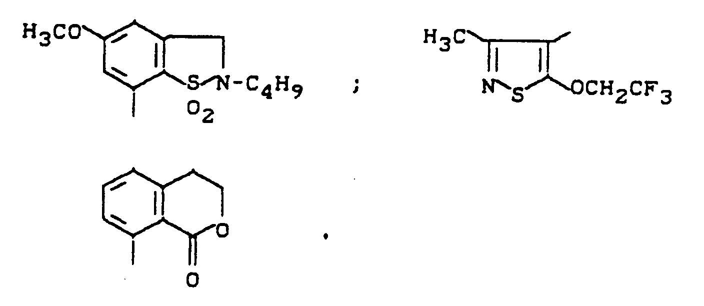
and salts of compounds of the formula (I).

2. Compounds of the formula (I) according to Claim 1, characterised in that, in these formulae,
R¹ represents hydrogen, C₁-C₄-alkyl which is optionally substituted by fluorine, cyano, methoxy or ethoxy, or represents allyl, or represents C₃-C₆-cycloalkyl,
R² represents C₁-C₄-alkyl which is optionally substituted by fluorine and/or chlorine, methoxy or ethoxy, or represents C₃-C₄-alkenyl which is optionally substituted by fluorine and/or chlorine, or represents C₃-C₆-cycloalkyl, and
R³ represents the group 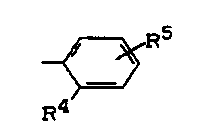 where
R⁴ represents fluorine, chlorine, bromine, methyl, trifluoromethyl, methoxy, difluoromethoxy, trifluoromethoxy, 2-chloro-ethoxy, 2-methoxy-ethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, methoxyaminosulphonyl, phenyl, phenoxy or C₁-C₃-alkoxy-carbonyl, and
R⁵ represents hydrogen, fluorine, chlorine or bromine;
furthermore
R³ represents the radical 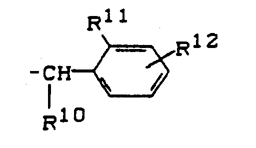 where
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 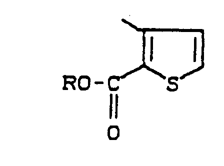 where R represents C₁-C₄-alkyl, or represents the radical 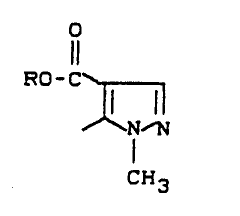 where R represents C₁-C₄-alkyl.

3. Process for the preparation of sulphonylaminocarbonyltriazolinones having two substituents bonded via oxygen of the general formula (I) according to Claim 1
and salts of compounds of the formula (I),
characterised in that
(a) triazolinones of the general formula (II) 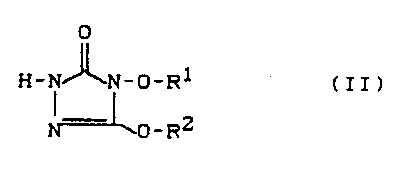 in which
R¹ and R² have the meanings given in Claim 1,
are reacted with sulphonyl isocyanates of the general formula (III)
R³-SO₂-N=C=O (III)
in which
R³ has the meaning given in Claim 1,
if appropriate in the presence of a diluent, or in that
(b) triazolinone derivatives of the general formula (IV) 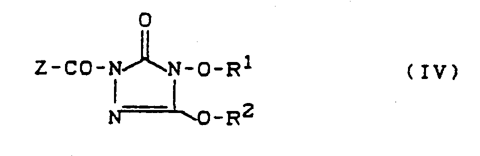 in which
R¹ and R² have the abovementioned meanings and
Z represents chlorine, C₁-C₄-alkoxy, benzyloxy or phenoxy,
are reacted with sulphonamides of the general formula (V)
R³-SO₂-NH₂ (V)
in which
R³ has the abovementioned meaning,
if appropriate in the presence of an acid acceptor and if appropriate in the presence of a diluent, or in that
(c) triazolinones of the general formula (II) 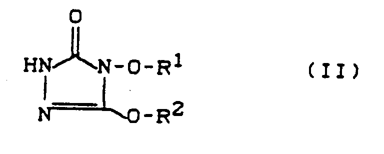 in which
R¹ and R² have the abovementioned meanings,
are reacted with sulphonamide derivatives of the general formula (VI)
R³-SO₂-NH-CO-Z (VI)
in which
R³ has the abovementioned meaning and
Z represents chlorine, C₁-C₄-alkoxy, benzyloxy or phenoxy,
if appropriate in the presence of an acid acceptor and if appropriate in the presence of a diluent,
and, if appropriate, salts are formed with the compounds of the formula (I) prepared by process (a), (b) or (c), using customary methods.

4. Herbicidal agents, characterised in that they contain at least one sulphonylaminocarbonyltriazolinone of the formula (I) according to Claim 1.

5. Use of sulphonylaminocarbonyltriazolinones of the general formula (I) according to Claim 1 for combating undesired plant growth.

6. Process for combating weeds, characterised in that sulphonylaminocarbonyltriazolinones of the general formula (I) according to Claim 1 are allowed to act on the weeds or their environment.

7. Process for the preparation of herbicidal agents, characterised in that sulphonylaminocarbonyltriazolinones of the general formula (I) according to Claim 1 are mixed with extenders and/or surface-active agents.

8. Triazolinones of the general formula (II) 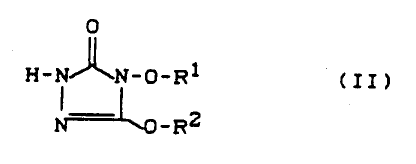 in which
R¹ represents hydrogen, or represents C₁-C₆-alkyl which is optionally substituted by fluorine, chlorine, cyano or C₁-C₄-alkoxy, or represents C₃-C₆-alkenyl which is optionally substituted by fluorine, chlorine and/or bromine, or represents C₃-C₇-cycloalkyl which is optionally substituted by fluorine, chlorine, bromine and/or C₁-C₄-alkyl,
R² represents C₁-C₆-alkyl which is optionally substituted by fluorine, chlorine, C₃-C₆-cycloalkyl or C₁-C₄-alkoxy or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl,
with the exception of the compounds 4-hydroxy-5-methoxy-2,4-dihydro-3H-1,2,4-triazol-3-one and 4-hydroxy-5-ethoxy-2,4-dihydro-3H-1,2,4-triazol-3-one.

9. Triazolinone derivatives of the general formula (IV) 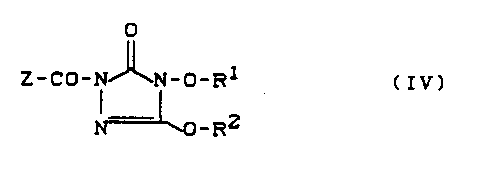 in which
R¹ represents hydrogen, or represents C₁-C₆-alkyl which is optionally substituted by fluorine, chlorine, cyano or C₁-C₄-alkoxy, or represents C₃-C₆-alkenyl which is optionally substituted by fluorine, chlorine and/or bromine, or represents C₃-C₇-cycloalkyl which is optionally substituted by fluorine, chlorine, bromine and/or C₁-C₄-alkyl,
R² represents C₁-C₆-alkyl which is optionally substituted by fluorine, chlorine, C₃-C₆-cycloalkyl or C₁-C₄-alkoxy or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl, and
Z represents chlorine, C₁-C₄-alkoxy, benzyloxy or phenoxy.

## Claims (Claims for the following Contracting State(s): ES)

1. Process for the preparation of sulphonylaminocarbonyltriazolinones having two substituents bonded via oxygen of the general formula (I) 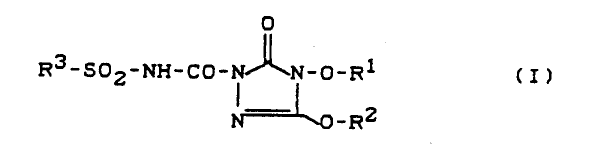 in which
R¹ represents hydrogen, or represents C₁-C₆-alkyl which is optionally substituted by fluorine, chlorine, cyano or C₁-C₄-alkoxy, or represents C₃-C₆-alkenyl which is optionally substituted by fluorine, chlorine and/or bromine, or represents C₃-C₇-cycloalkyl which is optionally substituted by fluorine, chlorine, bromine and/or C₁-C₄-alkyl,
R² represents C₁-C₆-alkyl which is optionally substituted by fluorine, chlorine, C₃-C₆-cycloalkyl or C₁-C₄-alkoxy or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl, and
R³ represents the group 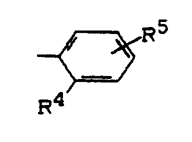 where
R⁴ and R⁵ are identical or different and represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)-aminosulphonyl, C₃-C₆-cycloalkyl or phenyl), or represents C₂-C₆-alkenyl (which is optionally substituted by fluorine, chlorine, bromine, cyano, C₁-C₄-alkoxy-carbonyl, carboxyl or phenyl), or represents C₂-C₆-alkinyl (which is optionally substituted by fluorine, chlorine, bromine, cyano, C₁-C₄-alkoxy-carbonyl, carboxyl or phenyl), or represents C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or represents C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl), or represents C₃-C₆-alkenyloxy (which is optionally substituted by fluorine, chlorine, bromine, cyano or C₁-C₄-alkoxy-carbonyl), or represents C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio, or the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl, or the radical -NHOR⁷, where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or represents benzhydryl, or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ furthermore represent phenyl or phenoxy, or represent C₁-C₄-alkylcarbonylamino, C₁-C₄-alkoxy-carbonylamino, C₁-C₄-alkylamino-carbonyl-amino, di-(C₁-C₄-alkyl)-amino-carbonylamino, or represent the radical -CO-R⁸, where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (each of which is optionally substituted by fluorine and/or chlorine),
R⁴ and R⁵ furthermore represent trimethylsilyl, thiazolinyl, C₁-C₄-alkylsulphonyloxy, di-(C₁-C₄-alkyl)-aminosulphonylamino or represent the radical -CH=N-R⁹, where
R⁹ represents C₁-C₆-alkyl which is optionally substituted by fluorine, chlorine, cyano, carboxyl, C₁-C₄-alkoxy, C₁-C₄-alkylthio, C₁-C₄-alkylsulphinyl or C₁-C₄-alkylsulphonyl, or represents benzyl which is optionally substituted by fluorine or chlorine, or represents C₃-C₆-alkenyl or C₃-C₆-alkinyl, each of which is optionally substituted by fluorine or chlorine, or represents phenyl which is optionally substituted by fluorine, chlorine, bromine, C₁-C₄-alkyl, C₁-C₄-alkoxy, trifluoromethyl, trifluoromethoxy or trifluoromethylthio, or represents C₁-C₆-alkoxy, C₃-C₆-alkenoxy, C₃-C₆-alkinoxy or benzyloxy, each of which is optionally substituted by fluorine and/or chlorine, or represents amino, C₁-C₄-alkylamino, di-(C₁-C₄-alkyl)-amino, phenylamino, C₁-C₄-alkyl-carbonyl-amino, C₁-C₄-alkoxy-carbonylamino, C₁-C₄-alkyl-sulphonylamino, or represents phenylsulphonylamino which is optionally substituted by fluorine, chlorine, bromine or methyl,
furthermore
R³ represents the radical 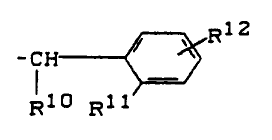 where
R¹⁰ represents hydrogen or C₁-C₄-alkyl,
R¹¹ and R¹² are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-amino sulphonyl;
furthermore
R³ represents the radical 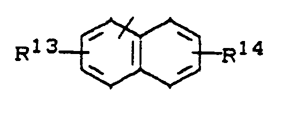 where
R¹³ and R¹⁴ are identical or different and represent hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 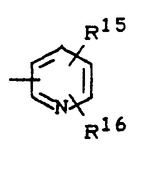 where
R¹⁵ and R¹⁶ are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent aminosulphonyl, mono-(C₁-C₄-alkyl)-aminosulphonyl, or represent di-(C₁-C₄-alkyl)-aminosulphonyl or C₁-C₄-alkoxycarbonyl or dimethylaminocarbonyl;
furthermore
R³ represents the radical 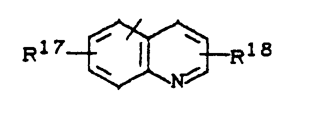 where
R¹⁷ and R¹⁸ are identical or different and represent hydrogen, fluorine, chlorine, bromine, C₁-C₄-alkyl (which is optionally substituted by fluorine and/or bromine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent di-(C₁-C₄-alkyl)-aminosulphonyl;
furthermore
R³ represents the radical 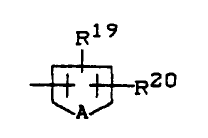 where
R¹⁹ and R²⁰ are identical or different and represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-aminosulphonyl, C₁-C₄-alkoxy-carbonyl or dimethylaminocarbonyl, and
A represents oxygen, sulphur or the group N-Z¹, where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di-(C₁-C₄-alkyl)-aminocarbonyl;
furthermore
R³ represents the radical 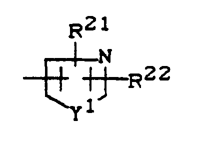 where
R²¹ and R²² are identical or different and represent hydrogen, C₁-C₄-alkyl, halogen, C₁-C₄-alkoxy-carbonyl, C₁-C₄-alkoxy or C₁-C₄-halogenoalkoxy,
Y¹ represents sulphur or the group N-R²³, where
R²³ represents hydrogen or C₁-C₄-alkyl;
furthermore
R³ represents the radical 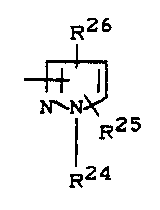 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl, and
R²⁶ represents hydrogen, halogen or C₁-C₄-alkyl;
furthermore
R³ represents one of the groups mentioned below, 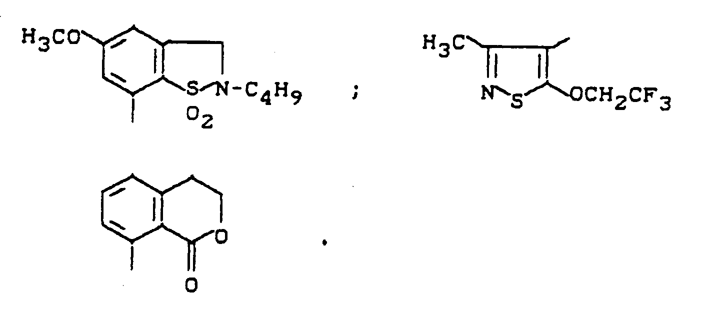
and salts of compounds of the formula (I),
characterised in that
(a) triazolinones of the general formula (II) 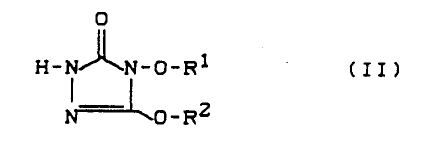 in which
R¹ and R² have the abovementioned meanings,
are reacted with sulphonyl isocyanates of the general formula (III)
R³-SO₂-N=C=O (III)
in which
R³ has the abovementioned meaning,
if appropriate in the presence of a diluent, or in that
(b) triazolinone derivatives of the general formula (IV) 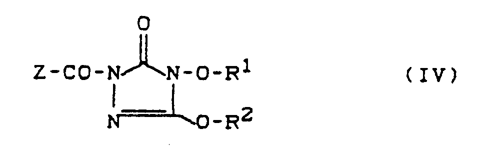 in which
R¹ and R² have the abovementioned meanings and
Z represents chlorine, C₁-C₄-alkoxy, benzyloxy or phenoxy,
are reacted with sulphonamides of the general formula (V)
R³-SO₂-NH₂ (V)
in which
R³ has the abovementioned meaning,
if appropriate in the presence of an acid acceptor and if appropriate in the presence of a diluent, or in that
(c) triazolinones of the general formula (II) 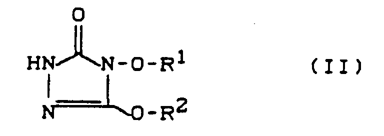 in which
R¹ and R² have the abovementioned meanings,
are reacted with sulphonamide derivatives of the general formula (VI)
R³-SO₂-NH-CO-Z (VI)
in which
R³ has the abovementioned meaning and
Z represents chlorine, C₁-C₄-alkoxy, benzyloxy or phenoxy,
if appropriate in the presence of an acid acceptor and if appropriate in the presence of a diluent,
and, if appropriate, salts are formed with the compounds of the formula (I) prepared by process (a), (b) or (c), using customary methods.

2. Herbicidal agents, characterised in that they contain at least one sulphonylaminocarbonyltriazolinone of the formula (I) according to Claim 1.

3. Use of sulphonylaminocarbonyltriazolinones of the general formula (I) according to Claim 1 for combating undesired plant growth.

4. Process for combating weeds, characterised in that sulphonylaminocarbonyltriazolinones of the general formula (I) according to Claim 1 are allowed to act on the weeds or their environment.

5. Process for the preparation of herbicidal agents, characterised in that sulphonylaminocarbonyltriazolinones of the general formula (I) according to Claim 1 are mixed with extenders and/or surface-active agents.

## Revendications (Revendications pour l'(les) Etat(s) contractant(s) suivant(s): BE, CH, DE, FR, GB, IT, LI, NL)

1. Sulfonylaminocarbonyltriazolinones portant deux substituants qui y sont liés par de l'oxygène, de formule générale (I) 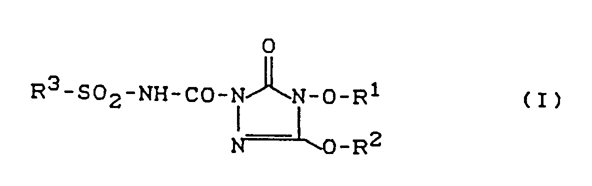 dans laquelle
R¹ représente de l'hydrogène, un groupe alkyle en C₁ à C₆ éventuellement substitué par du fluor, du chlore, un radical cyano ou alkoxy en C₁ à C₄, un groupe alcényle en C₃ à C₆ éventuellement substitué par du fluor, du chlore et/ou du brome ou un groupe cycloalkyle en C₃ à C₇ éventuellement substitué par du fluor, du chlore, du brome et/ou un radical alkyle en C₁ à C₄,
R² représente un groupe alkyle en C₁ à C₆ éventuellement substitué par du fluor, du chlore, un radical cycloalkyle en C₃ à C₆ ou alkoxy en C₁ à C₄, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un radical alkyle en C₁ à C₄, ou un groupe cyclohexényle et
R³ représente le groupe 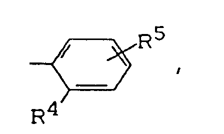 dans lequel
R⁴ et R⁵ sont identiques ou différents et représentent de l'hydrogène, du fluor, du chlore, du brome, de l'iode, un groupe nitro, un groupe alkyle en C₁ à C₆ (qui est substitué le cas échéant par du fluor, du chlore, du brome, un radical cyano, carboxy, (alkoxy en C₁ à C₄)-carbonyle, (alkylamino en C₁ à C₄)-carbonyle, di(alkyle en C₁ à C₄)amino-carbonyle, hydroxy, alkoxy en C₁ à C₄, formyloxy, (alkyle en C₁ à C₄)carbonyloxy, (alkoxy en C₁ à C₄)carbonyloxy, (alkylamino en C₁ à C₄)carbonyloxy, alkylthio en C₁ à C₄, alkylsulfinyle en C₁ à C₄, alkylsulfonyle en C₁ à C₄, di(alkyle en C₁ à C₄)amino-sulfonyle, cycloalkyle en C₃ à C₆ ou phényle), un groupe alcényle en C₂ à C₆ (qui est substitué le cas échéant par du fluor, du chlore, du brome, un radical cyano, (alkoxy en C₁ à C₄)carbonyle, carboxy ou phényle), un groupe alcynyle en C₂ à C₆ (qui est substitué le cas échéant par du fluor, du chlore, du brome, un radical cyano, (alkoxy en C₁ à C₄)carbonyle, carboxy ou phényle), un groupe alkoxy en C₁ à C₄ (qui est substitué le cas échéant par du fluor, du chlore, du brome, un radical cyano, carboxy, (alkoxy en C₁ à C₄)carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est substitué le cas échéant par du fluor, du chlore, du brome, un radical cyano, carboxy, (alkoxy en C₁ à C₄)-carbonyle, alkylthio en C₁ à C₄, alkylsulfinyle en C₁ à C₄ ou alkylsulfonyle en C₁ à C₄), un groupe alcényloxy en C₃ à C₆ (qui est substitué le cas échéant par du fluor, du chlore, du brome, un radical cyano ou (alkoxy en C₁ à C₄)-carbonyle), un groupe alcénylthio en C₂ à C₆ (qui est substitué le cas échéant par du fluor, du chlore, du brome, un radical cyano, nitro, alkylthio en C₁ à C₃ ou (alkoxy en C₁ à C₄)-carbonyle), un groupe alcynyloxy en C₃ à C₆, alcynylthio en C₃ à C₆ ou le reste -S(O)ₚ-R⁶, dans lequel
p représente le nombre 1 ou 2 et
R⁶ est un groupe alkyle en C₁ à C₄ (qui est substitué le cas échéant par du fluor, du chlore, du brome, un radical cyano ou (alkoxy en C₁ à C₄)-carbonyle), un groupe alcényle en C₃ à C₆, alcynyle en C₃ à C₆, alkoxy en C₁ à C₄, (alkoxy en C₁ à C₄)-alkylamino en C₁ à C₄, alkylamino en C₁ à C₄, di(alkyle en C₁ à C₄)amino, phényle ou le reste -NHOR⁷, dans lequel
R⁷ est un groupe alkyle en C₁ à C₁₂ (qui est substitué le cas échéant par du fluor, du chlore, un radical cyano, alkoxy en C₁ à C₄, alkylthio en C₁ à C₄, alkylsulfinyle en C₁ à C₄, alkylsulfonyle en C₁ à C₄), (alkyle en C₁ à C₄)carbonyle, (alkoxy en C₁ à C₄)carbonyle, (alkylamino en C₁ à C₄)carbonyle ou di(alkyle en C₁ à C₄)aminocarbonyle), un groupe alcényle en C₃ à C₆ (qui est substitué le cas échéant par du fluor, du chlore ou du brome), un groupe alcynyle en C₃ à C₆, cycloalkyle en C₃ à C₆, (cycloalkyle en C₃ à C₆)-alkyle en C₁ ou C₂, phényl-(alkyle en C₁ ou C₂) (qui est substitué le cas échéant par du fluor, du chlore, un radical nitro, cyano, alkyle en C₁ à C₄, alkoxy en C₁ à C₄ ou (alkoxy en C₁ à C₄)carbonyle), un groupe benzhydryle ou un groupe phényle (qui est substitué le cas échéant par du fluor, du chlore, un radical nitro, cyano, alkyle en C₁ à C₄, trifluorométhyle, alkoxy en C₁ à C₄, fluoralkoxy en C₁ ou C₂, alkylthio en C₁ à C₄, trifluorométhylthio, ou (alkoxy en C₁ à C₄)-carbonyle),
R⁴ et/ou R⁵ représentent en outre un groupe phényle ou un groupe phénoxy, un groupe (alkyle en C₁ à C₄)-carbonylamino, (alkoxy en C₁ à C₄)-carbonylamino, (alkylamino en C₁ à C₄)-carbonylamino, di(alkyle en C₁ à C₄)-aminocarbonylamino, ou le reste -CO-R⁸, dans lequel
R⁸ est un groupe alkyle en C₁ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-(alkylamino en C₁ à C₄) ou di(alkyle en C₁ à C₄)amino (qui sont substitués le cas échéant par du fluor et/ou du chlore),
R⁴ et/ou R⁵ représentent en outre un groupe triméthylsilyle, thiazolinyle, alkylsulfonyloxy en C₁ à C₄, di(alkyle en C₁ à C₄)aminosulfonylamino ou le reste -CH=N-R⁹ dans lequel
R⁹ représente un groupe alkyle en C₁ à C₆ éventuellement substitué par du fluor, du chlore, un radical cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou alcynyle en C₃ à C₆ éventuellement substitué par du fluor ou du chlore, un groupe phényle éventuellement substitué par du fluor, du chlore, du brome, un radical alkyle en C₁ à C₄, alkoxy en C₁ à C₄, trifluorométhyle, trifluorométhoxy ou trifluorométhylthio, un groupe alkoxy en C₁ à C₆, alcénoxy en C₃ à C₆, alcynoxy en C₃ à C₆ ou benzyloxy éventuellement substitué par du fluor et/ou du chlore, un groupe amino, alkylamino en C₁ à C₄, di(alkyle en C₁ à C₄)amino, phénylamino, (alkyle en C₁ à C₄)carbonylamino, (alkoxy en C₁ à C₄)carbonylamino, alkylsulfonylamino en C₁ à C₄ ou un groupe phénylsulfonylamino éventuellement substitué par du fluor, du chlore, du brome ou un radical méthyle,
en outre
R³ représente le reste 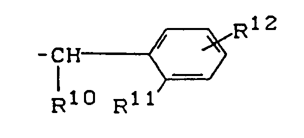 dans lequel
R¹⁰ est de l'hydrogène ou un groupe alkyle en C₁ à C₄
R¹¹ et R¹² sont identiques ou différents et représentent de l'hydrogène, du fluor, du chlore, du brome, un groupe nitro, cyano, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe carboxy, (alkoxy en C₁ à C₄)carbonyle, diméthylaminocarbonyle, alkylsulfonyle en C₁ à C₄ ou di(alkyle en C₁ à C₄)-aminosulfonyle,
en outre
R³ représente le reste 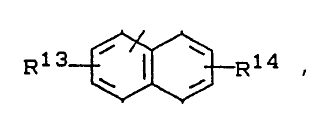 dans lequel
R¹³ et R¹⁴ sont identiques ou différents et représentent de l'hydrogène, du fluor, du chlore, du brome, un groupe nitro, cyano, alkyle en C₁ à C₄ (qui est substitué le cas échéant par du fluor et/ou du chlore) ou alkoxy en C₁ à C₄ (qui est substitué le cas échéant par du fluor et/ou du chlore) ;
en outre
R³ représente le reste 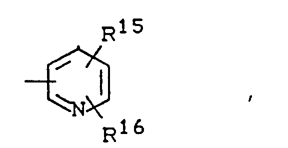 dans lequel
R¹⁵ et R¹⁶ sont identiques ou différents et représentent de l'hydrogène, du fluor, du chlore, du brome, un groupe nitro, cyano, alkyle en C₁ à C₄ (qui est substitué le cas échéant par du fluor et/ou du chlore), alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui sont substitués le cas échéant par du fluor et/ou du chlore), un groupe aminosulfonyle, mono(alkyle en C₁ à C₄)aminosulfonyle, di(alkyle en C₁ à C₄)aminosulfonyle ou (alkoxy en C₁ à C₄)carbonyle ou diméthylaminocarbonyle ;
en outre
R³ représente le reste 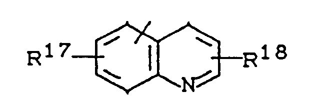 dans lequel
R¹⁷ et R¹⁸ sont identiques ou différents et représentent de l'hydrogène, du fluor, du chlore, du brome, un groupe alkyle en C₁ à C₄ (qui est substitué le cas échéant par du fluor et/ou du brome), un groupe alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui est substitué le cas échéant par du fluor et/ou du chlore), ou un groupe di(alkyle en C₁ à C₄)aminosulfonyle ;
en outre
R³ représente le reste 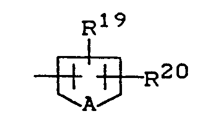 dans lequel
R¹⁹ et R²⁰ sont identiques ou différents et représentent de l'hydrogène, du fluor, du chlore, du brome, un groupe cyano, nitro, alkyle en C₁ à C₄ (qui est substitué le cas échéant par du fluor et/ou du chlore), alkoxy en C₁ à C₄ (qui est substitué le cas échéant par du fluor et/ou du chlore), alkylthio en C₁ à C₄, alkylsulfinyle en C₁ à C₄ ou alkylsulfonyle en C₁ à C₄ (qui sont substitués le cas échéant par du fluor et/ou du chlore), un groupe di(alkyle en C₁ à C₄)aminosulfonyle, (alkoxy en C₁ à C₄)-carbonyle ou diméthylaminocarbonyle et
A représente de l'oxygène, du soufre ou le groupe N-Z¹ dans lequel
Z¹ est de l'hydrogène, un groupe alkyle en C₁ à C₄ (qui est substitué le cas échéant par du fluor, du chlore, du brome ou un radical cyano), un groupe cycloalkyle en C₃ à C₆, benzyle, phényle (qui est substitué le cas échéant par du fluor, du chlore, du brome ou un radical nitro), un groupe (alkyle en C₁ à C₄)carbonyle, (alkoxy en C₁ à C₄)-carbonyle ou di(alkyle en C₁ à C₄)aminocarbonyle ;
en outre
R³ représente le reste 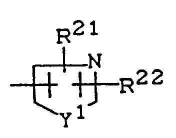 dans lequel
R²¹ et R²² sont identiques ou différents et représentent de l'hydrogène, un groupe alkyle en C₁ à C₄, un halogène, un groupe alkoxy en C₁ à C₄)-carbonyle, alkoxy en C₁ à C₄ ou halogénalkoxy en C₁ à C₄,
Y¹ représente du soufre ou le groupe N-R²³ dans lequel
R²³ est de l'hydrogène ou un groupe alkyle en C₁ à C₄ ;
en outre
R³ représente le reste 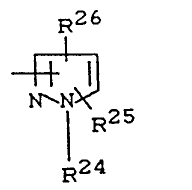 dans lequel
R²⁴ est de l'hydrogène, un groupe alkyle en C₁ à C₄, benzyle, pyridyle, quinolyle ou phényle,
R²⁵ est de l'hydrogène, un halogène, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe dioxolanyle ou (alkoxy en C₁ à C₄)carbonyle et
R²⁶ est de l'hydrogène, un halogène ou un groupe alkyle en C₁ à C₄ ;
en outre
R³ représente l'un des groupements indiqués ci-après : 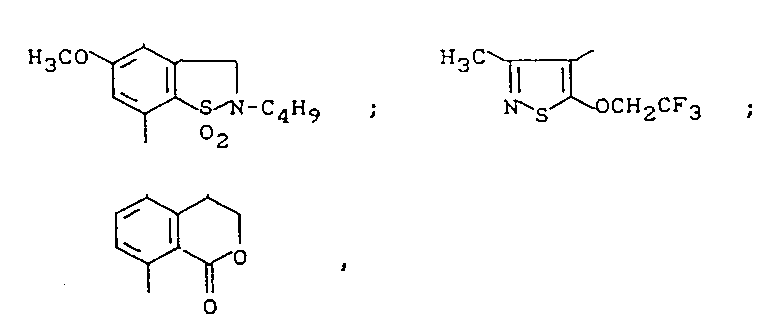
ainsi que des sels de composés de formule (I).

2. Composés de formule (I) suivant la revendication 1, caractérisés en ce que, dans leur formule :
R¹ représente de l'hydrogène, un groupe alkyle en C₁ à C₄ éventuellement substitué par du fluor, un radical cyano, méthoxy ou éthoxy, un groupe allyle ou un groupe cycloalkyle en C₃ à C₆,
R² est un groupe alkyle en C₁ à C₄ éventuellement substitué par du fluor et/ou du chlore, un radical méthoxy ou éthoxy, un groupe alcényle en C₃ ou C₄ éventuellement substitué par du fluor et/ou du chlore ou un groupe cycloalkyle en C₃ à C₆, et
R³ représente le groupement 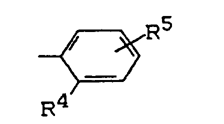 dans lequel
R⁴ est du fluor, du chlore, du brome, un groupe méthyle, trifluorométhyle, méthoxy, difluorométhoxy, trifluorométhoxy, 2-chloréthoxy, 2-méthoxyéthoxy, alkylthio en C₁ à C₃, alkylsulfinyle en C₁ à C₃, alkylsulfonyle en C₁ à C₃, diméthylamino-sulfonyle, diéthylaminosulfonyle, N-méthoxy-N-méthylaminosulfonyle, méthoxyaminosulfonyle, phényle, phénoxy ou (alkoxy en C₁ à C₃)-carbonyle et
R⁵ est de l'hydrogène, du fluor, du chlore ou du brome ;
en outre
R³ représente le reste 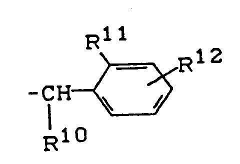 dans lequel
R¹⁰ est de l'hydrogène,
R¹¹ est du fluor, du chlore, du brome, un groupe méthyle, méthoxy, difluorométhoxy, trifluorométhoxy, éthoxy, méthoxycarbonyle, éthoxycarbonyle, méthylsulfonyle ou diméthylaminosulfonyle et
R¹² est de l'hydrogène ;
en outre
R³ représente le reste 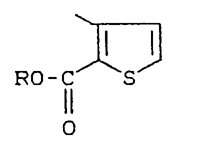 dans lequel R est un groupe alkyle en C₁ à C₄,
ou bien 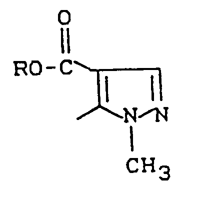 le reste
dans lequel R est un groupe alkyle en C₁ à C₄.

3. Procédé de production de sulfonylaminocarbonyltriazolinones portant deux substituants qui y sont liés par l'intermédiaire d'oxygène, de formule générale (I) suivant la revendication 1, ainsi que de sels de composés de formule (I), caractérisé en ce que
(a) on fait réagir des triazolinones de formule générale (II) 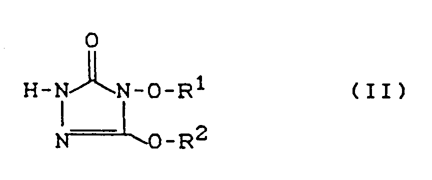 dans laquelle
R¹ et R² ont les définitions indiquées dans la revendication 1,
avec des sulfonylisocyanates de formule générale (III)
R³-SO₂-N=C=O (III)
dans laquelle
R³ a la définition indiquée dans la revendication 1,
le cas échéant en présence d'un diluant, ou bien
(b) on fait réagir des dérivés de triazolinones de formule générale (IV) 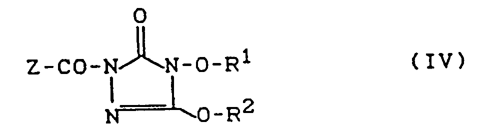 dans laquelle
R¹ et R² ont les définitions indiquées ci-dessus et
Z représente du chlore, un groupe alkoxy en C₁ à C₄, benzyloxy ou phénoxy,
avec des amides d'acides sulfoniques de formule générale (V)
R³-SO₂-NH₂ (V)
dans laquelle
R³ a la définition indiquée ci-dessus,
le cas échéant en présence d'un accepteur d'acide et en la présence éventuelle d'un diluant, ou bien
(c) on fait réagir des triazolinones de formule générale (II) 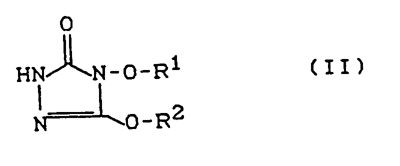 dans laquelle
R¹ et R² ont les définitions indiquées ci-dessus,
avec des dérivés d'amides d'acides sulfoniques de formule générale (VI)
R³-SO₂-NH-CO-Z (VI)
dans laquelle
R³ a la définition indiquée ci-dessus et
Z représente du chlore, un groupe alkoxy en C₁ à C₄, benzyloxy ou phénoxy,
le cas échéant en présence d'un accepteur d'acide et en la présence éventuelle d'un diluant,
et le cas échéant, on produit des sels par des opérations classiques à partir des composés de formule (I) préparés selon les procédés (a), (b) ou (c).

4. Compositions herbicides, caractérisées par une teneur en au moins une sulfonylaminocarbonyltriazolinone de formule (I) suivant la revendication 1.

5. Utilisation de sulfonylaminocarbonyltriazolinones de formule générale (I) suivant la revendication 1 pour combattre la croissance non désirée de plantes.

6. Procédé pour combattre des mauvaises herbes, caractérisé en ce qu'on fait agir des sulfonylaminocarbonyltriazolinones de formule générale (I) suivant la revendication 1 sur les mauvaises herbes ou sur leur milieu.

7. Procédé de préparation de compositions herbicides, caractérisé en ce qu'on mélange des sulfonylaminocarbonyltriazolinones de formule générale (I) suivant la revendication 1 avec des diluants et/ou des agents tensioactifs.

8. Triazolinones de formule générale (II) 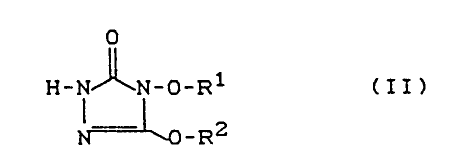 dans laquelle
R¹ représente de l'hydrogène, un groupe alkyle en C₁ à C₆ éventuellement substitué par du fluor, du chlore, un radical cyano ou alkoxy en C₁ à C₄, un groupe alcényle en C₃ à C₆ éventuellement substitué par du fluor, du chlore et/ou du brome ou un groupe cycloalkyle en C₃ à C₇ éventuellement substitué par du fluor, du chlore, du brome et/ou un radical alkyle en C₁ à C₄ et
R² représente un groupe alkyle en C₁ à C₆ éventuellement substitué par du fluor, du chlore, un radical cycloalkyle en C₃ à C₆ ou alkoxy en C₁ à C₄, un groupe alcényle en C₃ à C₆ ou alcynyle en C₃ à C₆ éventuellement substitué par du fluor, du chlore et/ou du brome, un groupe cycloalkyle en C₃ à C₆ éventuellement substitué par du fluor, du chlore, du brome et/ou un radical alkyle en C₁ à C₄ ou un groupe cyclohexényle,
excepté les composés 4-hydroxy-5-méthoxy-2,4-dihydro-3H-1,2,4-triazole-3-one et 4-hydroxy-5-éthoxy-2,4-dihydro-3H-1,2,4-triazole-3-one.

9. Dérivés de triazolinones de formule générale (IV) 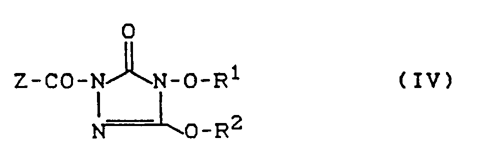 dans laquelle
R¹ représente de l'hydrogène, un groupe alkyle en C₁ à C₆ éventuellement substitué par du fluor, du chlore, un radical cyano ou alkoxy en C₁ à C₄, un groupe alcényle en C₃ à C₆ éventuellement substitué par du fluor, du chlore et/ou du brome ou un groupe cycloalkyle en C₃ à C₇ éventuellement substitué par du fluor, du chlore, du brome et/ou un radical alkyle en C₁ à C₄,
R² est un groupe alkyle en C₁ à C₆ éventuellement substitué par du fluor, du chlore, un radical cycloalkyle en C₃ à C₆ ou alkoxy en C₁ à C₄, un groupe alcényle en C₃ à C₆ ou alcynyle en C₃ à C₆ éventuellement substitué par du fluor, du chlore et/ou du brome, un groupe cycloalkyle en C₃ à C₆ éventuellement substitué par du fluor, du chlore, du brome et/ou un radical alkyle en C₁ à C₄, ou un groupe cyclohexényle et
Z représente du chlore, un groupe alkoxy en C₁ à C₄, benzyloxy ou phénoxy.

## Revendications (Revendications pour l'(les) Etat(s) contractant(s) suivant(s): ES)

1. Procédé de production de sulfonylaminocarbonyltriazolinones portant deux substituants qui y sont liés par l'intermédiaire d'oxygène, de formule générale (I) 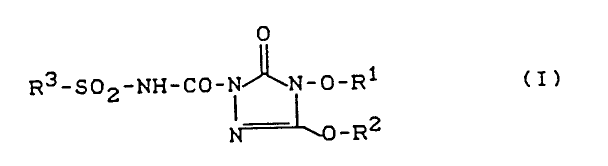 dans laquelle
R¹ représente de l'hydrogène, un groupe alkyle en C₁ à C₆ éventuellement substitué par du fluor, du chlore, un radical cyano ou alkoxy en C₁ à C₄, un groupe alcényle en C₃ à C₆ éventuellement substitué par du fluor, du chlore et/ou du brome ou un groupe cycloalkyle en C₃ à C₇ éventuellement substitué par du fluor, du chlore, du brome et/ou un radical alkyle en C₁ à C₄,
R² représente un groupe alkyle en C₁ à C₆ éventuellement substitué par du fluor, du chlore, un radical cycloalkyle en C₃ à C₆ ou alkoxy en C₁ à C₄, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un radical alkyle en C₁ à C₄, ou un groupe cyclohexényle et
R³ représente le groupe 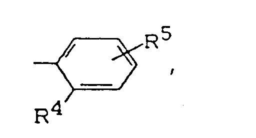 dans lequel
R⁴ et R⁵ sont identiques ou différents et représentent de l'hydrogène, du fluor, du chlore, du brome, de l'iode, un groupe nitro, un groupe alkyle en C₁ à C₆ (qui est substitué le cas échéant par du fluor, du chlore, du brome, un radical cyano, carboxy, (alkoxy en C₁ à C₄)-carbonyle, (alkylamino en C₁ à C₄)-carbonyle, di(alkyle en C₁ à C₄)amino-carbonyle, hydroxy, alkoxy en C₁ à C₄, formyloxy, (alkyle en C₁ à C₄)carbonyloxy, (alkoxy en C₁ à C₄)carbonyloxy, (alkylamino en C₁ à C₄)carbonyloxy, alkylthio en C₁ à C₄) alkylsulfinyle en C₁ à C₄, alkylsulfonyle en C₁ à C₄, di(alkyle en C₁ à C₄)amino-sulfonyle, cycloalkyle en C₃ à C₆ ou phényle), un groupe alcényle en C₂ à C₆ (qui est substitué le cas échéant par du fluor, du chlore, du brome, un radical cyano, (alkoxy en C₁ à C₄)carbonyle, carboxy ou phényle), un groupe alcynyle en C₂ à C₆ (qui est substitué le cas échéant par du fluor, du chlore, du brome, un radical cyano, (alkoxy en C₁ à C₄)carbonyle, carboxy ou phényle), un groupe alkoxy en C₁ à C₄ (qui est substitué le cas échéant par du fluor, du chlore, du brome, un radical cyano, carboxy, (alkoxy en C₁ à C₄)carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est substitué le cas échéant par du fluor, du chlore, du brome, un radical cyano, carboxy, (alkoxy en C₁ à C₄)-carbonyle, alkylthio en C₁ à C₄, alkylsulfinyle en C₁ à C₄ ou alkylsulfonyle en C₁ à C₄), un groupe alcényloxy en C₃ à C₆ (qui est substitué le cas échéant par du fluor, du chlore, du brome, un radical cyano ou (alkoxy en C₁ à C₄)-carbonyle), un groupe alcénylthio en C₂ à C₆ (qui est substitué le cas échéant par du fluor, du chlore, du brome, un radical cyano, nitro, alkylthio en C₁ à C₃ ou (alkoxy en C₁ à C₄)-carbonyle), un groupe alcynyloxy en C₃ à C₆, alcynylthio en C₃ à C₆ ou le reste -S(O)ₚ-R⁶, dans lequel
p représente le nombre 1 ou 2 et
R⁶ est un groupe alkyle en C₁ à C₄ (qui est substitué le cas échéant par du fluor, du chlore, du brome, un radical cyano ou (alkoxy en C₁ à C₄)-carbonyle), un groupe alcényle en C₃ à C₆, alcynyle en C₃ à C₆, alkoxy en C₁ à C₄, (alkoxy en C₁ à C₄)-alkylamino en C₁ à C₄, alkylamino en C₁ à C₄, di(alkyle en C₁ à C₄)amino, phényle ou le reste -NHOR⁷, dans lequel
R⁷ est un groupe alkyle en C₁ à C₁₂ (qui est substitué le cas échéant par du fluor, du chlore, un radical cyano, alkoxy en C₁ à C₄, alkylthio en C₁ à C₄, alkylsulfinyle en C₁ à C₄, alkylsulfonyle en C₁ à C₄), (alkyle en C₁ à C₄)carbonyle, (alkoxy en C₁ à C₄)carbonyle, (alkylamino en C₁ à C₄)carbonyle ou di(alkyle en C₁ à C₄)aminocarbonyle), un groupe alcényle en C₃ à C₆ (qui est substitué le cas échéant par du fluor, du chlore ou du brome), un groupe alcynyle en C₃ à C₆, cycloalkyle en C₃ à C₆, (cycloalkyle en C₃ à C₆)-alkyle en C₁ ou C₂, phényl-(alkyle en C₁ ou C₂) (qui est substitué le cas échéant par du fluor, du chlore, un radical nitro, cyano, alkyle en C₁ à C₄, alkoxy en C₁ à C₄ ou (alkoxy en C₁ à C₄)carbonyle), un groupe benzhydryle ou un groupe phényle (qui est substitué le cas échéant par du fluor, du chlore, un radical nitro, cyano, alkyle en C₁ à C₄, trifluorométhyle, alkoxy en C₁ à C₄, fluoralkoxy en C₁ ou C₂, alkylthio en C₁ à C₄, trifluorométhylthio, ou (alkoxy en C₁ à C₄)-carbonyle),
R⁴ et/ou R⁵ représentent en outre un groupe phényle ou un groupe phénoxy, un groupe (alkyle en C₁ à C₄)-carbonylamino, (alkoxy en C₁ à C₄)-carbonylamino, (alkylamino en C₁ à C₄)-carbonylamino, di(alkyle en C₁ à C₄)-aminocarbonylamino, ou le reste -CO-R⁸, dans lequel
R⁸ est un groupe alkyle en C₁ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-(alkylamino en C₁ à C₄) ou di(alkyle en C₁ à C₄)amino (qui sont substitués le cas échéant par du fluor et/ou du chlore),
R⁴ et/ou R⁵ représentent en outre un groupe triméthylsilyle, thiazolinyle, alkylsulfonyloxy en C₁ à C₄, di(alkyle en C₁ à C₄)aminosulfonylamino ou le reste -CH=N-R⁹ dans lequel
R⁹ représente un groupe alkyle en C₁ à C₆ éventuellement substitué par du fluor, du chlore, un radical cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou alcynyle en C₃ à C₆ éventuellement substitué par du fluor ou du chlore, un groupe phényle éventuellement substitué par du fluor, du chlore, du brome, un radical alkyle en C₁ à C₄, alkoxy en C₁ à C₄, trifluorométhyle, trifluorométhoxy ou trifluorométhylthio, un groupe alkoxy en C₁ à C₆, alcénoxy en C₃ à C₆, alcynoxy en C₃ à C₆ ou benzyloxy éventuellement substitué par du fluor et/ou du chlore, un groupe amino, alkylamino en C₁ à C₄, di(alkyle en C₁ à C₄)amino, phénylamino, (alkyle en C₁ à C₄)carbonylamino, (alkoxy en C₁ à C₄)carbonylamino, alkylsulfonylamino en C₁ à C₄ ou un groupe phénylsulfonylamino éventuellement substitué par du fluor, du chlore, du brome ou un radical méthyle,
en outre
R³ représente le reste 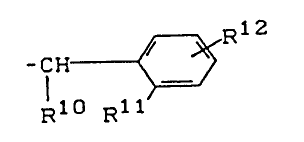 dans lequel
R¹⁰ est de l'hydrogène ou un groupe alkyle en C₁ à C₄
R¹¹ et R¹² sont identiques ou différents et représentent de l'hydrogène, du fluor, du chlore, du brome, un groupe nitro, cyano, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe carboxy, (alkoxy en C₁ à C₄)carbonyle, diméthylaminocarbonyle, alkylsulfonyle en C₁ à C₄ ou di(alkyle en C₁ à C₄)-aminosulfonyle,
en outre
R³ représente le reste 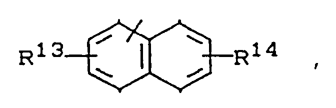 dans lequel
R¹³ et R¹⁴ sont identiques ou différents et représentent de l'hydrogène, du fluor, du chlore, du brome, un groupe nitro, cyano, alkyle en C₁ à C₄ (qui est substitué le cas échéant par du fluor et/ou du chlore) ou alkoxy en C₁ à C₄ (qui est substitué le cas échéant par du fluor et/ou du chlore) ;
en outre
R³ représente le reste 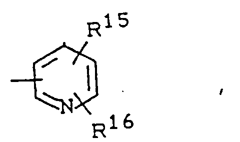 dans lequel
R¹⁵ et R¹⁶ sont identiques ou différents et représentent de l'hydrogène, du fluor, du chlore, du brome, un groupe nitro, cyano, alkyle en C₁ à C₄ (qui est substitué le cas échéant par du fluor et/ou du chlore), alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui sont substitués le cas échéant par du fluor et/ou du chlore), un groupe aminosulfonyle, mono(alkyle en C₁ à C₄)aminosulfonyle, di(alkyle en C₁ à C₄)aminosulfonyle ou (alkoxy en C₁ à C₄)carbonyle ou diméthylaminocarbonyle ;
en outre
R³ représente le reste 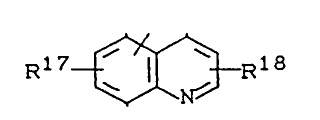 dans lequel
R¹⁷ et R¹⁸ sont identiques ou différents et représentent de l'hydrogène, du fluor, du chlore, du brome, un groupe alkyle en C₁ à C₄ (qui est substitué le cas échéant par du fluor et/ou du brome), un groupe alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui est substitué le cas échéant par du fluor et/ou du chlore), ou un groupe di(alkyle en C₁ à C₄)aminosulfonyle ;
en outre
R³ représente le reste 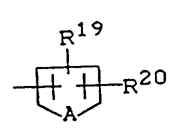 dans lequel
R¹⁹ et R²⁰ sont identiques ou différents et représentent de l'hydrogène, du fluor, du chlore, du brome, un groupe cyano, nitro, alkyle en C₁ à C₄ (qui est substitué le cas échéant par du fluor et/ou du chlore), alkoxy en C₁ à C₄ (qui est substitué le cas échéant par du fluor et/ou du chlore), alkylthio en C₁ à C₄, alkylsulfinyle en C₁ à C₄ ou alkylsulfonyle en C₁ à C₄ (qui sont substitués le cas échéant par du fluor et/ou du chlore), un groupe di(alkyle en C₁ à C₄)aminosulfonyle, (alkoxy en C₁ à C₄)-carbonyle ou diméthylaminocarbonyle et
A représente de l'oxygène, du soufre ou le groupe N-Z¹ dans lequel
Z¹ est de l'hydrogène, un groupe alkyle en C₁ à C₄ (qui est substitué le cas échéant par du fluor, du chlore, du brome ou un radical cyano), un groupe cycloalkyle en C₃ à C₆, benzyle, phényle (qui est substitué le cas échéant par du fluor, du chlore, du brome ou un radical nitro), un groupe (alkyle en C₁ à C₄)carbonyle, (alkoxy en C₁ à C₄)-carbonyle ou di(alkyle en C₁ à C₄)aminocarbonyle ;
en outre
R³ représente le reste 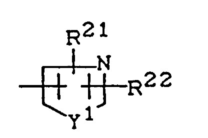 dans lequel
R²¹ et R²² sont identiques ou différents et représentent de l'hydrogène, un groupe alkyle en C₁ à C₄, un halogène, un groupe alkoxy en C₁ à C₄)-carbonyle, alkoxy en C₁ à C₄ ou halogénalkoxy en C₁ à C₄,
Y¹ représente du soufre ou le groupe N-R²³ dans lequel
R²³ est de l'hydrogène ou un groupe alkyle en C₁ à C₄ ;
en outre
R³ représente le reste 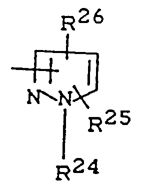 dans lequel
R²⁴ est de l'hydrogène, un groupe alkyle en C₁ à C₄, benzyle, pyridyle, quinolyle ou phényle,
R²⁵ est de l'hydrogène, un halogène, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe dioxolanyle ou (alkoxy en C₁ à C₄)carbonyle et
R²⁶ est de l'hydrogène, un halogène ou un groupe alkyle en C₁ à C₄ ;
en outre
R³ représente l'un des groupements indiqués ci-après : 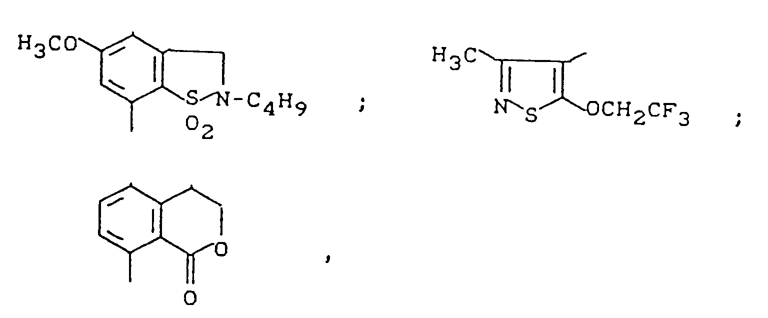
ainsi que des sels de composés de formule (I),
caractérisé en ce que :
(a) on fait réagir des triazolinones de formule générale (II) 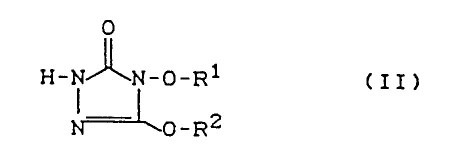 dans laquelle
R¹ et R² ont les définitions indiquées ci-dessus,
avec des sulfonylisocyanates de formule générale (III)
R³-SO₂-N=C=O (III)
dans laquelle
R³ a la définition indiquée ci-dessus,
le cas échéant en présence d'un diluant, ou bien
(b) on fait réagir des dérivés de triazolinones de formule générale (IV) 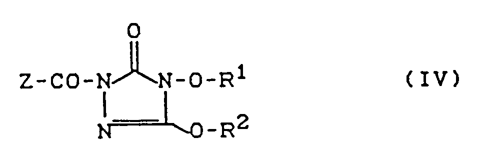 dans laquelle
R¹ et R² ont les définitions indiquées ci-dessus et
Z représente du chlore, un groupe alkoxy en C₁ à C₄, benzyloxy ou phénoxy,
avec des amides d'acides sulfoniques de formule générale (V)
R³-SO₂-NH₂ (V)
dans laquelle
R³ a la définition indiquée ci-dessus,
le cas échéant en présence d'un accepteur d'acide et en la présence éventuelle d'un diluant, ou bien
(c) on fait réagir des triazolinones de formule générale (II) 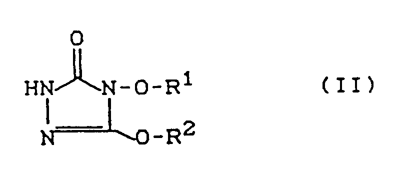 dans laquelle
R¹ et R² ont les définitions indiquées ci-dessus,
avec des dérivés d'amides d'acides sulfoniques de formule générale (VI)
R³-SO₂-NH-CO-Z (VI)
dans laquelle
R³ a la définition indiquée ci-dessus et
Z représente du chlore, un groupe alkoxy en C₁ à C₄, benzyloxy ou phénoxy,
le cas échéant en présence d'un accepteur d'acide et en la présence éventuelle d'un diluant,
et le cas échéant, on produit des sels par des opérations classiques à partir des composés de formule (I) préparés selon les procédés (a), (b) ou (c).

2. Compositions herbicides, caractérisées par une teneur en au moins une sulfonylaminocarbonyltriazolinone de formule (I) suivant la revendication 1.

3. Utilisation de sulfonylaminocarbonyltriazolinones de formule générale (I) suivant la revendication 1 pour combattre la croissance non désirée de plantes.

4. Procédé pour combattre les mauvaises herbes, caractérisé en ce qu'on fait agir des sulfonylaminocarbonyltriazolinones de formule générale (I) suivant la revendication 1 sur les mauvaises herbes ou sur leur milieu.

5. Procédé de préparation de compositions herbicides, caractérisé en ce qu'on mélange des sulfonylaminocarbonyltriazolinones de formule générale (I) suivant la revendication 1 avec des diluants et/ou des agents tensioactifs.
